# EUROPEAN PATENT APPLICATION

(11) **EP 4 074 725 A2**
(43) Date of publication of application: **19.10.2022**
(21) Application number: 20898023.5
(22) Date of filing: 02.12.2020
(51) Int. Cl.: C07K 7/08, G01N 33/53, G01N 33/58, A61K 47/62, G01N 33/544, G01N 33/68

(54) **SWITCHING BINDER, PREPARATION METHOD THEREFOR, AND PHARMACEUTICAL COMPOSITION, ASSAY KIT, AND ANTIGEN AND ANTIBODY ASSAY METHOD, EACH USING SAME**

(30) Priority: 09.12.2019 KR 20190162707; 17.06.2020 KR 20200073508; 23.10.2020 KR 20200138705
(71) Applicant: Optolane Technologies Inc., Bundang-gu Seongnam-si, Gyeonggi-do 13494 (KR)
(72) Inventor: SHIN, Seung-Shick, Gwangju-si Gyeonggi-do 12793 (KR); LEE, Do young, Seoul 06002 (KR); SEO, Seong-Min, Seongnam-si Gyeonggi-do 13494 (KR); CHOI, Kyung-Hak, Yongin-si Gyeonggi-do 17000 (KR); PYUN, Jae-Chul, Seoul 06521 (KR)
(74) Representative: Schiweck Weinzierl Koch Patentanwälte Partnerschaft mbB
(86) International application number: PCT/KR2020/017497
(87) International publication number: WO 2021/118156

(57) **Abstract**

The present invention relates to a switching binder binding to an antigen-binding portion of an antibody, a preparation method thereof, and a pharmaceutical composition, an assay kit, and an antigen and antibody assay method, each utilizing the same. A switching binder according to an embodiment of the present invention includes an amino acid sequence selected from framework regions of a heavy chain or a light chain, which constitutes an antigen-binding portion of an antibody, wherein the amino acid sequence includes, in addition to the heavy chain or the light chain, a first antibody-binding portion or a second antibody-binding portion that binds to a framework region of a heavy chain or a light chain, which constitutes the antigen-binding portion, the amino acid sequence being able to bind specifically to the antigen-binding portion of the antibody and then be separated from the framework region when a target antigen is provided.

【Sequence Listing Free Text】

<110>	OPTOLANE TECHNOLOGIES INC.

<120>	Switching binder, method of fabricating the same, and pharmaceutical
composition, assay kit, and method of analyzing antigen and antibody using the same

<130>	IN00300PCT

<150>	KR 10-2019-0162707

<151>	2019-12-09

<150>	KR 10-2020-0073508

<151>	2020-06-17

<150>	KR 10-2020-0138705

<151>	2020-10-23

<160>	4

<170>	KoPatentIn 3.0

<210>	1

<211>	20

<212>	PRT

<213>	Artificial Sequence

<220>

<223>	peptide 1

<400>	1

Ser Tyr Trp Ile His Trp Val Lys Gln Arg Pro Gly Gln Gly Leu Glu

1               5                   10                  15

Trp Ile Gly Glu

            20

<210>	2

<211>	20

<212>	PRT

<213>	Artificial Sequence

<220>

<223>	peptide 2

<400>	2

Val Tyr Tyr Cys Ala Arg Glu Pro Thr Gly Thr Gly Ile Tyr Phe Asp

1               5                   10                  15

Val Trp Gly Lys

            20

<210>	3

<211>	20

<212>	PRT

<213>	Artificial Sequence

<220>

<223>	peptide 3

<400>	3

Thr Tyr Leu Glu Trp Tyr Pro Gln Lys Pro Gly Gln Ser Pro Lys Leu

1               5                   10                  15

Leu Ile Tyr Lys

            20

<210>	4

<211>	14

<212>	PRT

<213>	Artificial Sequence

<220>

<223>	peptide 4

<400>	4

Val Tyr Tyr Cys Phe Gln Gly Ser His Val Pro Phe Thr Lys

1               5                   10

## Description

### [Technical Field]

The present disclosure relates to immunoassay technology, and more specifically, the present disclosure relates to a switching binder, a preparation method thereof, and a pharmaceutical composition, an assay kit, and an antigen and antibody assay method, each using the same.

### [Background Art]

Immunoassay (or immunological test) has been used for bioassays such as various immunodiagnostic tests or environmental monitoring in the field of biotechnology. The immunoassay is a method for detecting or quantifying an antigen or antibody using the high specificity of antigen-antibody reaction. Either one of or both the antigen and antibody are labeled by various methods and then reacted with a sample. Thereafter, if there is an antibody or antigen in the sample, the antigen-antibody reaction is generated, so the labeled product can be measured to qualitatively or quantitatively measure the antibody or antigen. This has the advantage of having high sensitivity compared to other analytical methods.

The immunoassay can be used for various diagnoses, such as cancer markers, infectious diseases, thyroid function, anemia, allergies, pregnancy, drug abuse, or gout diagnosis. In order to use the immunoassay for diagnosis or analysis of various types of antigens, it is necessary to prepare antibodies having specific reactivity with respect to each of the antigens. However, in recent years, this method has limitations in detecting modern diseases that are rapidly diversifying or viruses with multiple mutant species. In particular, in the case of viruses, there exist many cases where it is impossible to secure an antibody having specific binding to the viruses.

In addition, when using the antigen-antibody reaction, an additional step of binding the antibody to a labeling material may be required. If the binding property between the labeling material and the antibody is low, the accuracy or reliability of the immunoassay may be reduced. As described above, the conventional immunoassay has an additional problem in that various types of labeling materials capable of binding to multiple antibodies should be developed.

### [Disclosure]

### [TECHNICAL PROBLEM]

One aspect of the present disclosure is to provide a switching binder with improved applicability without being limited to the type of antibody as it can detect various types of diseases or antigens that cause the diseases.

Another aspect of the present disclosure is to provide a method for preparing a switching binder having the above-mentioned advantages.

Still another aspect of the present disclosure is to provide a test kit with improved reliability and accuracy of analysis by using a switching binder having the above-described advantages. In addition, a test kit capable of measuring the binding strength of an antigen to an antibody is provided.

Still yet another aspect of the present disclosure is to provide an assay method that has the above-described advantages, improves analysis reliability and accuracy, and simplifies processing steps for analysis, thereby improving speed and ease of use.

### [Technical Solution]

To this end, the switching binder according to an embodiment of the present disclosure may reversibly bind to at least a portion of a framework region (FR) of an antibody and be separated from the framework region when a target antigen that specifically reacts with the antibody binds to the antibody. According to other embodiment, the switching binder may bind to the framework region by three-dimensional structure of the antibody. According to another embodiment, the switching binder may include a moiety capable of binding to at least one framework region and a moiety capable of binding to at least one complementarity determining region (CDR).

According to an embodiment, the switching binder may include an amino acid sequence selected from a framework region of a heavy chain constituting an antigen-binding site of an antibody, wherein the amino acid sequence may include a first antibody-binding portion reversibly bound to a framework region of a light chain constituting the antigen-binding site together with the heavy chain. According to other embodiment, the switching binder may include an amino acid sequence selected from the framework region of a light chain constituting an antigen-binding site of an antibody, wherein the amino acid sequence may include a second antibody-binding portion reversibly bound to a framework region of a heavy chain constituting the antigen-binding site together with the light chain. According to another embodiment, the amino acid sequence of the switching binder may also reversibly bind to a complementarity determining region adjacent to the framework region of the heavy chain or the light chain.

According to an embodiment, the switching binder may be selected from the group consisting of a peptide, a protein, an aptamer, a nanobody, a phage display, a yeast display, and an antibody. According to other embodiment, a portion of the amino acid of the switching binder may be substituted with an inorganic material.

According to an embodiment, the first antibody-binding portion may include at least one amino acid residue of -Y-, -I-, -W-, and -Q-. According to other embodiment, the second antibody-binding portion may include at least one amino acid residue of -Y-, -Q-, -P-, -L-, and -F-. According to another embodiment, the switching binder may include any one of the amino acid sequences of SEQ ID NO. 1: S-Y-W-I-H-W-V-K-Q-R-P-G-Q-G-L-E-W-I-G-E, SEQ ID NO. 2: V-Y-Y-C-A-R-E-P-T-G-T-G-I-Y-F-D-V-W-G-K, SEQ ID NO. 3: T-Y-L-E-W-Y-P-Q-K-P-G-Q-S-P-K-L-L-I-Y-K, and SEQ ID NO. 4: V-Y-Y-C-F-Q-G-S-H-V-P-F-T-K.

According to an embodiment, the first antibody-binding portion may include an amino acid residue that binds to the framework region of the light chain by at least one selected from electrostatic interaction, hydrogen bond, van der Waals force, hydrophobic interaction, and combinations thereof. According to other embodiment, the second antibody-binding portion may include an amino acid residue that binds to the framework region of the heavy chain by at least one selected from electrostatic interaction, hydrogen bond, van der Waals force, hydrophobic interaction, and combinations thereof.

According to an embodiment, the switching binder may be capable of regulating the binding strength of the antibody-binding site by amino acid modulation of the first antibody-binding portion. According to other embodiment, the switching binder may be capable of regulating the binding strength of the antibody-binding site by amino acid modulation of the second antibody-binding portion. According to another embodiment, the switching binder may be bound to a side chain functional group bond or a polar region of a peptide bond between the switching binder and the framework region by the hydrogen bond.

According to an embodiment, when the switching binder is bound to the antigen-binding portion of the antibody, the switching binder may be separated from the antibody by binding a target antigen that specifically reacts with the antibody to the antibody. According to other embodiment, the antibody-binding portion of the switching binder may be bound to the antigen-binding portion of the heavy chain or the light chain within a range of 4 Å to 8 Å.

According to an embodiment, the switching binder may be labeled with a natural or artificial labeling material. According to an embodiment, the switching binder binds to a human antibody, a non-human antibody, a humanized non-human antibody, or a combination thereof, and in a state in which the switching binder is bound to the antibody, the binding of the antibody and the target antigen may be possible.

According to an embodiment, the switching binder may include a first antibody-binding portion or a second antibody-binding portion reversibly and specifically binding to at least some of the framework regions (FRs) of the antibody; and a binding strength regulating portion for adjusting a binding strength of the first antibody-binding portion or the second antibody-binding portion to the framework regions such that the antibody-binding portion is separated from the framework regions when the target antigen that specifically reacts with the antibody binds to the antibody. In other embodiment, the binding strength regulating portion may modulate the position of the first antibody-binding portion or the second antibody-binding portion in the switching binder or the three-dimensional shape of the switching binder to adjust the binding strength of the antibody-binding portion to the antibody.

According to an embodiment, the binding strength regulating portion may include at least one amino acid sequence selected from -S-Y-W-I-H-W-V-K-, -R-P-G-Q-G-L-E-, -I-G-E-, -V-Y-, -C-A-R-E-P-T-G-T-G-, -Y-F-D-V-, -G-K-, -T-Y-L-E-W-Y-, -K-P-G-Q-S-, -K-, L-I-Y-K-, -C-F-Q-G-S-H-V-P-, and -T-K-. According to other embodiment, with the inclusion of the first antibody-binding portion or the second antibody-binding portion and the binding strength regulating portion as a unit structure, the antibody-binding portion and the binding strength regulating portion may be alternately coupled to each other consecutively.

A pharmaceutical composition for disease screening, diagnosis, prophylaxis, or treatment integration according to an embodiment of the present disclosure for solving the above problems may include the switching binder. According to an embodiment, the switching binder in the pharmaceutical composition may be selected from the group consisting of a peptide, a protein, an aptamer, a nanobody, a phage display, a yeast display, and an antibody. According to other embodiment, the switching binder in the pharmaceutical composition may include any one of the amino acid sequences of SEQ ID NO. 1: S-Y-W-I-H-W-V-K-Q-R-P-G-Q-G-L-E-W-I-G-E, SEQ ID NO. 2: V-Y-Y-C-A-R-E-P-T-G-T-G-I-Y-F-D-V-W-G-K, SEQ ID NO. 3: T-Y-L-E-W-Y-P-Q-K-P-G-Q-S-P-K-L-L-I-Y-K, and SEQ ID NO. 4: V-Y-Y-C-F-Q-G-S-H-V-P-F-T-K.

A test kit according to an embodiment of the present disclosure for solving the above problems includes the switching binder, wherein the switching binder is labeled with a natural or artificial labeling material, and the binding strength of the antigen to the antibody can be measured by measuring the amount of the switching binder bound to and dissociated from the framework regions. According to an embodiment, the switching binder may be selected from the group consisting of a peptide, a protein, an aptamer, a nanobody, a phage display, a yeast display, and an antibody. According to other embodiment, the binding strength may be measured by calculating the antigen-binding coefficient (K_{d}) of the antibody. According to another embodiment, the antigen-binding coefficient (K_{d}) may include a range of 0.7 × 10⁻⁶ M to 3.4 × 10⁻⁶ M.

According to an embodiment, the test kit includes the switching binder, wherein the switching binder is labeled with a natural or artificial labeling material, and an antibody and a target antigen can be identified or quantified by measuring an amount of the switching binder bound to and dissociated from the framework regions. According to other embodiment, the test kit is an immunoassay kit, wherein the immunoassay kit comprises a luminex assay, a protein microarray assay, an ELISA assay, an immuno-PCR assay, a capture-ELISA assay, an ELISPOT assay, a radioimmunoassay (RIA), a lateral flow immunoassay (LFIA), a flow-through immunoassay (FTIA), a protein microfluidic immunoassay, a protein capillary electrophoresis (CE) assay, an electrochemical biosensor, a radioimmunoprecipitation assay, an immunoprecipitation assay, an immunohistochemical staining assay, an Ouchterlony immunodiffusion assay, an inhibition or competition assay, a sandwich assay, a flow cytometry, an immunoelectrophoretic assay, a tissue immunostaining assay, a complement fixation assay, a FACS assay, a protein chip assay, an immunofluorescence staining assay, and an immunoaffinity purification assay kit. According to an embodiment, the test kit may measure the antigen-binding coefficient (K_{d}) when the antibody binds to an antigen in a solution to which the antibody is not immobilized.

The assay method using the switching binder according to an embodiment of the present disclosure for solving the above problems may include the steps of selecting an amino acid sequence of the switching binder from framework regions (FRs) constituting an antigen-binding site of an antibody; a pretreating to reversibly bind to at least a portion of the framework regions of the antibody by providing the switching binder to the antibody that specifically binds to a target antigen; providing an analyte that requires determination of whether the target antigen is included in the result of the pretreatment step; and analyzing to identify or quantify the target antigen in the analyte by quantifying at least one of a residual amount of the switching binder bound to the framework regions or the switching binder separated from the framework regions. According to other embodiment, the switching binder may be selected from the group consisting of a peptide, a protein, an aptamer, a nanobody, a phage display, a yeast display, and an antibody. According to another embodiment, the antibody may be immobilized on a support such as a substrate, a bead, a fiber, a polymer structure, or a combination thereof. According to still yet another embodiment, the antibody is not immobilized, but may be dissolved in a solution.

According to an embodiment, the pretreatment step may include the steps of providing the switching binder to the antibody to obtain a mixed solution comprising a first conjugate of the antibody and the switching binder and a switching binder not bound to the antibody, and removing the switching binder not bound to the antibody. According to an embodiment, the switching binder may be labeled with a natural or artificial labeling material, and in the analysis step, the analysis of the target antigen may be performed by measuring the labeling reaction. The analysis step may further include the steps of separating the switching binder separated from the antibody and a second conjugate of the antibody and the target antigen, and identifying or quantifying the separated switching binder.

A method for preparing the switching binder according to an embodiment of the present disclosure to solve the above problems may include the steps of identifying an amino acid sequence in which the framework regions of the heavy chain and light chain of the antibody self-bind with each other; obtaining a hydrophobicity index of the amino acid sequence; and comparing the hydrophobicity index, such that a switching binder comprising the amino acid sequence of a portion having the same property between the amino acid sequences of the framework regions constituting an antigen-binding site of the heavy chain and the light chain is selected. According to an embodiment, the amino acid sequence may include a complementarity determining region adjacent to the framework regions. According to an embodiment, the switching binder may be selected from the group consisting of a peptide, a protein, an aptamer, a nanobody, a phage display, a yeast display, and an antibody. According to other embodiment, the amino acid sequence may be bound by at least one selected from electrostatic interaction, hydrogen bond, van der Waals force, hydrophobic interaction, and combinations thereof.

### [Advantageous Effects]

A switching binder according to an embodiment of the present disclosure may be selected from an antigen-binding portion of an antibody, in particular, the framework regions having the same or substantially similar amino acid sequence for each antibody. As a result, there are advantages in that the switching binder can bind to various types of antibodies, can be used for analysis for identification or quantification of the antibodies and antigens bound to the antibodies regardless of the types of antibodies, and can modulate a binding strength for binding to the antibodies through modulation of the selected amino acid sequence. In addition, there are advantages in that the switching binder can bind to various types of antibodies and can be used for analysis for identification or quantification of antibodies and antigens bound to the antibodies regardless of the types of antibodies, and modulate a binding strength to the antibodies.

A method for preparing the switching binder according to an embodiment of the present disclosure has an advantage capable of preparing a switching binder that can bind to various antibodies, since it may be extracted from an antigen-binding portion of an antibody, particularly, framework regions.

A pharmaceutical composition according to an embodiment of the present disclosure for virus or disease screening, diagnosis, prophylaxis, or treatment has an advantage capable of using the switching binder having the above-described advantages and being developed into medications for diagnosis, prophylaxis, or treatment of antigens including various viruses.

A test kit according to an embodiment of the present disclosure can use the switching agent having the above-described advantages and can identify and quantify antigen and antibody as well as measure the antigen's binding ability to the antibody.

An assay method using the switching binder according to an embodiment of the present disclosure can omit the step of washing an analyte such as an antibody or antigen, or adding reagent treatment by using the switching binder having the above-described advantages, thereby quickly analyzing an antigen-binding and an amount of the bind, and providing improved use of immunoassay with ease.

### [Description of Drawings]

FIG. 1A is a diagram showing the combination of a switching binder extracted from framework regions and the framework regions according to an embodiment of the present disclosure, and FIG. 1B is a diagram showing the configuration of a switching binder according to an embodiment of the present disclosure.
FIG. 2 is a diagram showing the coupling of a switching binder to a framework region according to other embodiment of the present disclosure.
FIG. 3 is a diagram illustrating a switching binder according to an embodiment of the present disclosure.
FIG. 4A is a flow diagram of an assay method using a switching binder according to an embodiment of the present disclosure, FIG. 4B is a flow diagram of an assay method using a switching binder according to other embodiment of the present disclosure, and FIG. 4C is a flow diagram of an assay method using a switching binder according to still another embodiment of the present disclosure.
FIGS. 5A and 5B are diagrams showing the binding between the switching binder of SEQ ID NO. 2 and the switching binder of SEQ ID NO. 3; FIG. 5C is a graph showing the interaction between the switching binder of SEQ ID NO. 2 and the switching binder of SEQ ID NO. 3 for each site; FIG. 5D is a diagram showing the hydrophobicity index between the switching binder of SEQ ID NO. 2 and the switching binder of SEQ ID NO. 3; and FIG. 5E is a graph of FRET analysis between the switching binder of SEQ ID NO. 2 (H2) and the switching binder of SEQ ID NO. 3 (L1).
FIGS. 6A and 6B are diagrams showing the binding between the switching binder of SEQ ID NO. 1 and the switching binder of SEQ ID NO. 4; FIG. 6C is a graph showing the interaction between the switching binder of SEQ ID NO. 1 and the switching binder of SEQ ID NO. 4 for each site; FIG. 6D is a diagram showing the hydrophobicity index between the switching binder of SEQ ID NO. 1 and the switching binder of SEQ ID NO. 4; and FIG. 6E is a graph of FRET analysis between the switching binder of SEQ ID NO. 1 (H1) and the switching binder of SEQ ID NO. 4 (L2).
FIG. 7A is a diagram showing the measurement result of the antibody-binding strength for each switching binder; FIG. 7B is a diagram showing the results of SPR analysis for the binding strength of each switching binder bound to an antibody; FIGS. 7C and 7D are diagrams showing the results of binding to the antibody Fab site of a peptide bound to an antibody.
FIGS. 8A to 8D are graphs illustrating assay results according to an assay method of a switching binder according to an embodiment of the present disclosure.
FIGS. 9A and 9B are graphs illustrating assay results according to an assay method of a switching binder according to other embodiment of the present disclosure.
FIGS. 10A to 10D are graphs illustrating assay results according to an assay method of a switching binder according to still another embodiment of the present disclosure.

### [DESCRIPTION OF EMBODIMENTS]

Exemplary embodiments will now be described more fully with reference to the accompanying drawings.

Embodiments of the present disclosure are provided to more completely explain the present disclosure to those of ordinary skill in the art, and the following embodiments may be modified in various other forms, and the scope of the present disclosure is not limited to these embodiments. Rather, these embodiments are provided so that this disclosure will be more thorough and complete, and will fully convey the spirit of the present disclosure to those of ordinary skill in the art.

In the drawings, like reference numerals refer to like elements. As used herein, the term "and/or" includes any one and all combinations of one or more of those listed items.

The terminology used herein is used to describe the embodiment and is not intended to limit the scope of the present disclosure. Although the singular is used herein, the plural form may be included unless the context clearly indicates the plurality. As used herein, the terms "comprise" and/or "comprising" specify the presence of the recited shapes, numbers, steps, actions, members, elements, and/or groups thereof, but do not exclude the presence or addition of other shapes, numbers, movements, members, elements and/or groups.

Reference herein to a layer formed "on" a substrate or other layer refers to a layer formed directly on the substrate or other layer, or a layer formed on an intermediate layer or intermediate layers formed on the substrate or other layer. For those of skill in the art, a structure or shape disposed "adjacent" to another shape may have a portion disposed above or below the adjacent shape.

As used herein, the relative terms such as "below," "above," "upper," "lower," "horizontal," or "vertical" may be used to describe the relationship that one constituent member, layer or region has with another constituent member, layer or region, as shown in the drawings. It should be understood that these terms encompass not only the orientation indicated in the drawings, but also other orientations of the device.

Hereinafter, embodiments of the present disclosure will be described with reference to cross-sectional views schematically illustrating ideal embodiments (and intermediate structures) of the present disclosure. In these drawings, for example, the size and shape of the members may be exaggerated for convenience and clarity of description, and variations of the illustrated shape may be expected in actual implementation. Accordingly, embodiments of the present disclosure should not be construed as limited to the specific shapes of the regions shown herein. Reference numerals for members in the drawings refer to the same members throughout the drawings.

As used herein, the term "peptide" is an amino acid polymer chain and is an amide in which an amino group of the first amino acid and the carboxyl group of a second amino acid adjacent to the first amino acid are condensed while losing water molecules. All peptide sequences described herein are, by convention, written in an order with an N-terminal end to the left and a C-terminal end to the right. As used herein, the term "peptide" may refer to a natural or synthetic protein, protein fragment, or peptide analog of a protein sequence. The analog is defined as a substance exhibiting similar physical properties such as size, charge, or hydrophobicity exhibited by the corresponding basic amino acid or peptide in proper spatial orientation. For example, a peptide analog may be a compound in which an amide bond between one or more amino acids is replaced with a carbon-carbon bond or other bond known in the art. As used herein, the term "peptide" may be used interchangeably with the term "protein" or "polypeptide," which is a compound in which several amino acids are linked by peptide bonds and may refer to a molecular structure in which the number of amino acid residues is between 10 and 50 (inclusive).

As used herein, the term "amino acid" is a broad concept and includes not only naturally occurring amino acids and their residues, but also chemically modified amino acids, that is, amino acid mimetics and analogs, and the mimetics and analogs may include functional equivalents in the present disclosure.

As used herein, the term "aptamer" refers to a DNA nucleic acid molecule that has a stable tertiary structure by itself and can bind to a specific substance with high affinity and specificity. The aptamer may be prepared by a method well known in the art, and the method may be appropriately modified as necessary.

As used herein, the term "antigen-antibody reaction" is a specific chemical interaction between antibodies produced in B cells of leukocytes and antigens in the course of an immune response. Among different types of antibodies and antigens, each antibody can only bind to a specific antigen. The specificity of the binding is due to the specific chemical structure of each said antibody. The antigens bind to the antibody through weak noncovalent bonds such as electrostatic interactions, hydrogen bonds, van der Waals bonds, and hydrophobic interactions.

As used herein, the term "antibody" refers to a basic 4-polypeptide chain consisting of two heavy chains and two light chains, which are stabilized by disulfide bonds with each other and include any molecular construct comprising an antigen-binding moiety as a glycoprotein having the ability to specifically bind to an antigen.

The antibody may be a basic component of serum, an aggregate of the antibodies, immunoglobulin molecules, or fragments thereof, or a single molecule of an antibody or immunoglobulin. When the antibody molecule binds to an antigen or a specific antigenic determinant such as an epitope of the antigen, or the antibody binds to the antigen by reacting with the antigenic determinant, it is possible to induce immunological effector mechanisms such as antibody-dependent cellular cytotoxicity (ADCC) in which the antibody is recognized and eliminated by natural killer (NK) cells. The antibody may be monospecific. The composition of the antibodies may be monoclonal composed of the same antibodies, or polyclonal comprising different types of antibodies that react with different epitopes of the same antigen or with epitopes of different antigens. Each antibody may have a unique structure that allows for the specific binding of the antibody to its corresponding antigen.

The antibody may include an antibody, an antibody derivative, or a fragment thereof, and the detailed description of the antibody also applies to the antibody screening method of the present disclosure. Among antibody fragments, antibody equivalents or antibody homologs such as polypeptides include an Fc region, an immunoglobulin binding domain, a peptide mimicking a binding domain, a region homologous to the Fc region, or at least a portion thereof. Antibodies may also include chimeric molecules or equivalents thereof comprising an immunoglobulin binding domain fused to another polypeptide. Specifically, the antibodies may be intact immunoglobulin molecules, and include constituent parts of the antibody such as Fab, Fab', F(ab')2, Fc, VHH, Fv, N-glycan structure, and paratope, and at least some of the constituent parts.

The fragment antigen-binding (Fab) is an antigen-binding fragment comprising an antigen-binding portion and refers to an antibody fragment including a light chain fragment comprising a variable region (VL) and a constant region (CL) of a light chain, and a heavy chain fragment comprising a variable region (VH) and a first constant region (CH1).

The fragment crystallizable region (Fc region) corresponds to a portion that does not bind antigen but forms a crystal and generally refers to a dimer complex comprising the C-terminal polypeptide sequence of an immunoglobulin heavy chain. The Fc region may comprise a native or mutated Rc sequence. Although the Fc sequence in the heavy chain of an immunoglobulin may have multiple boundaries, the IgG heavy chain Fc sequence is usually defined as extending from an amino acid residue at position Cys226 or about position Pro230 to the carboxyl terminus of the Fc. The Fc sequence of an immunoglobulin generally comprises two constant regions of a heavy chain, a CH2 domain and a CH3 domain, and optionally a CH4 domain. For example, in a native antibody, the Fc domain consists of two identical protein fragments derived from CH2 and CH3 of the two heavy chains of the antibody in the IgG, IgA, and IgD isotypes, and the IgM and IgE Fc domains contain three heavy chain constant domains, CH2 to CH4, in each polypeptide chain. The Fc region can form crystals when proteins are gathered, which means that even if the antigen specificity of the IgG antibody is different, the amino acid sequence of the proteins constituting the Fc region is the same, and thus it is not involved in antigen binding. In addition, the Fc region forms a higher-order structure by -S-S- loops by using a certain number of amino acid residues as one division, and each division is linked by a peptide bond.

The Fragment variable (Fv) is a variable region of an antibody that directly binds to an antigen and consists of a heavy chain variable domain (V_{H}) and a light chain variable domain (L_{H}).

The V_{H}H is a single domain molecule without a light chain and is a single variable domain also called a nanobody. The naturally occurring single domain molecule may be derived or obtained from camelids such as camels, dromedaries, alpaca and guanaco, and sharks, and may be obtained by recombination of antibody molecules.

In the antibody, the heavy or light chain may include a variable region and a constant region, respectively. The variable region has specificity for an antigen and is different for each antibody. The variable region may include a hypervariable region (HVR), that is, complementarity determining regions (CDRs) and framework regions (FRs) supporting the complementarity determining regions. The complementarity determining regions (CDRs) have a different amino acid sequence for each antibody so that they can specifically bind to each antigen.

The framework region (FR) refers to constant domain residues other than hypervariable region residues. The framework region (FR) of the constant domain is generally comprised of four FR domains: FR1, FR2, FR3, and FR4, which are regions other than the CDRs present in the variable region and serve as a scaffold connecting the three CDRs and contribute to the structural stability of the CDRs. In addition, the amino acid sequence of the framework region (FR) has a characteristic that changes a little compared to the complementarity determining region (CDR) even if the type of antibody, that is, the expression species of the antibody is different. Thus, the CDR and FR sequences can generally appear in the variable region (V_{H}) of a heavy chain or a variable region (V_{L}) of a light chain in the following order: FR1-H1(L1)-FR2-H2(L2)-FR3-H3(L3)-FR4. The two variable regions of the heavy and light chains combine to form an antigen-binding portion.

The constant regions are substantially identical from antibody to antibody. The constant region, in general, is capable of mediating the binding of immunoglobulins to host tissues or factors, including components of the classical complement system, and various cells of the immune system such as effector cells, which are small molecules that selectively bind to proteins and modulate biological activity. Hereinafter, various embodiments of the present disclosure will be described in detail.

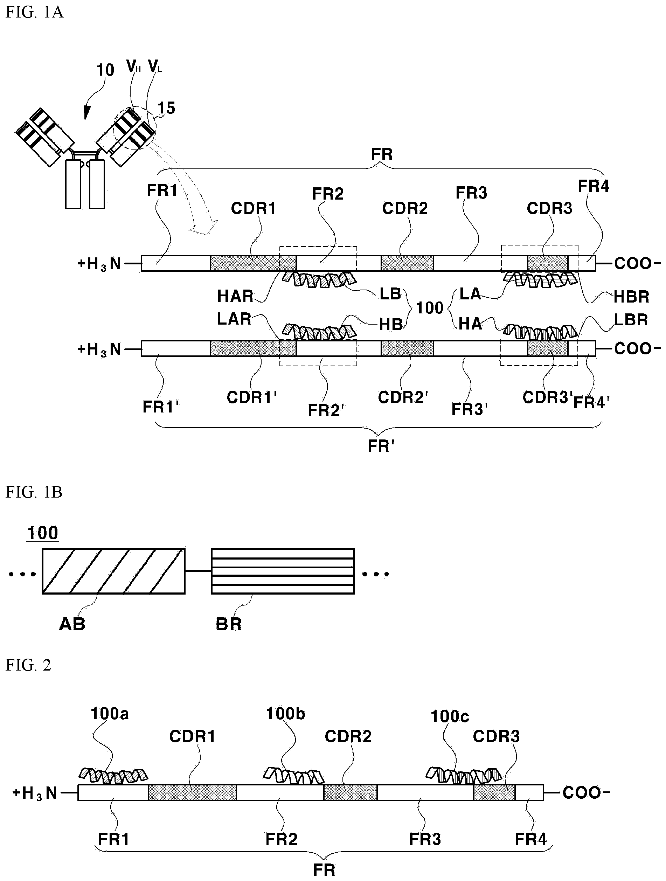

FIG. 1A shows that the switching binders 100 according to an embodiment of the present disclosure are bound to the framework regions (FR, FR') of the antibody 10.

Referring to FIG. 1A, the switching binder 100 includes a first switching binder (HA, HB) that reversibly binds to the framework region (FR') of the light chain (V_{L}) constituting the antigen-binding portion 15 of the antibody 10, or a second switching binder (LA, LB) that reversibly binds to the framework region (FR) of the heavy chain (V_{H}).

The first switching binder (HA, HB) may comprise an amino acid sequence selected from the framework region (FR) of the heavy chain (V_{H}) constituting the antigen-binding portion 15 of the antibody 10. Similarly, the second switching binder (LA, LB) may comprise an amino acid sequence selected from the framework region (FR') of the light chain (V_{L}) constituting the antigen-binding portion 15 of the antibody 10.

In another embodiment, the switching binder 100 may comprise an amino acid selected from an amino acid sequence comprising the framework regions (FR, FR') and a complementarity determining regions (CDR, CDR') adjacent to the framework regions (FR, FR'). For example, as shown in FIG. 1A, the amino acid sequence of the first switching binder (HA, HB) can be selected from an amino acid sequence comprising a part or all of the sequences of the CDR1 region (CDR1) of the complementarity determining regions (CDR) of the heavy chain (V_{H}) and the FR2 region (FR2) of the framework regions (FR)(hereinafter, referred to as a HAR region (HAR)), or an amino acid sequence comprising a part or all of the CDR3 region (CDR3) and the FR3 region (FR3) of the heavy chain (V_{H})(hereinafter referred to as an HBR region (HBR)).

Similarly, the amino acid sequence of the second switching binder (LA, LB) may be selected from an amino acid sequence comprising a part or all of the FR2' region (FR2') of the framework regions (FR') adjacent to each other of the light chain (V_{L}) and the CDR1' region (CDR1') of the adjacent complementarity determining regions (CDR')(hereinafter referred to as LAR region (LAR)), or an amino acid sequence comprising a part or all of the FR3' region (FR3') of the framework regions (FR') adjacent to each other of the light chain (V_{L}) and the CDR3' region (CDR3') of the adjacent complementarity determining regions (CDR')(hereinafter referred to as LBR region (LBR)).

The HAR region (HAR) and the LBR region (LBR) and the HBR region (HBR) and the LAR region (LAR) of the antibody 10 allow the antigen-binding portion 15 to have a three-dimensionally twisted structure since mutual self-binding or geometric cross-linking is possible. In addition, since the regions (HAR, LAR, HBR, LBR) have the framework regions (FR, FR') as essential components, the structure of the antibody 10 is supported through the above-described mutual binding properties. Since the switching binder 100 according to an embodiment of the present disclosure includes the amino acid sequence of the above regions of the antibody 10, self-binding or cross-linking to the antigen-binding portion of the antibody 10 is possible.

The switching binder 100 of the present disclosure is not limited to those selected from the HAR region (HAR), HBR region (HBR), LAR region (LAR), or LBR region (LBR) shown in FIG. 1A. It can be variously selected from the amino acid sequence of the framework regions (FRs). For example, the amino acid sequence of the switching binder 100 may be selected from the FR3 region (FR3) or FR3' region (FR3'), and a sequence used by linking the FR regions (FR) may also be used. In an embodiment, it is clear that an amino acid sequence is selected from the heavy chain (V_{H}) such as FR1 region (FR1), FR1 region (FR1) and CDR1 region (CDR1), FR2 region (FR2), FR1 region (FR1) to FR2 region (FR2) or FR2 region (FR2) to FR3 region (FR3), or the light chain (V_{L}) such as FR1' region (FR1'), FR1' region (FR1') and CDR1' region (CDR1'), FR2' region (FR2'), FR1' region (FR1') to FR2' region (FR2') or FR2' regions (FR2') to FR3' region (FR3'), as a site necessarily comprising at least one of the FR regions (FR, FR'). As such, the switching binder 100 is designed to comprise as a required configuration the amino acid sequence of the framework regions (FR, FR'), which is characterized by having the same or substantially similar sequence despite various antibodies, compared to the complementarity determining regions (CDR, CDR'), binding to the antigen-binding portion of the antibody is possible regardless of the types of antibodies or antigens. Various embodiments thereof will be described in detail with reference to FIG. 2.

The first switching binder (HA, HB) may reversibly bind to at least one of the framework regions (FR') of the light chain (V_{L}) due to the structural mutual binding properties of the antibody 10 described above. For example, the first switching binder (HA) may mutually bind to the LBR region (LBR), and the first switching binder (HB) may mutually bind to the LAR region (LAR). Similarly, the second switching binder (LA, LB) is capable of reversibly binding to at least one of the framework regions (FR) of the heavy chain (V_{H}). For example, the second switching binder (LA) may mutually bind to the HAR region (HAR), and the second switching binder (LB) may mutually bind to the HBR region (HBR). This allows the switching binder 100 to bind to the framework regions (FR, FR') constituting the antigen-binding portion 15 of the antibody 10 to support the structure of the antigen-binding portion 15 of the antibody 10.

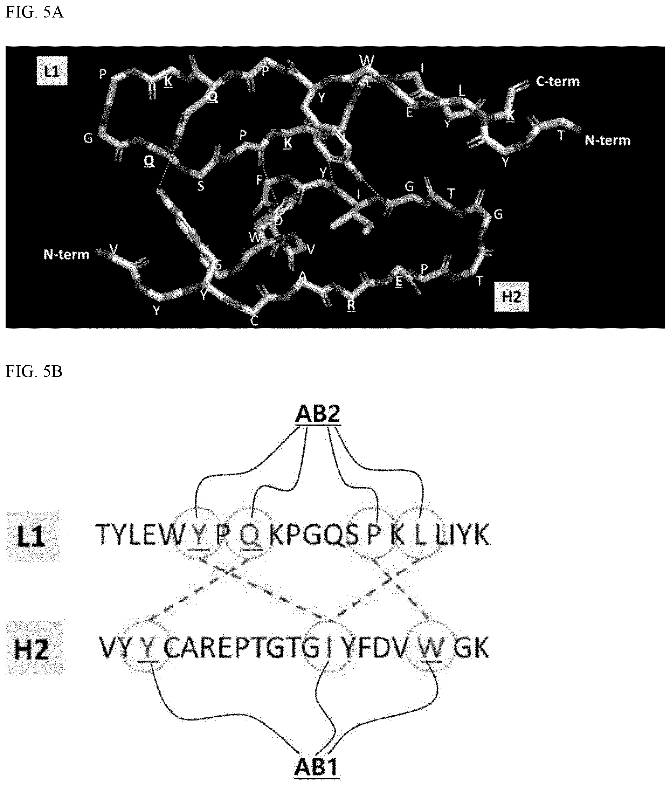

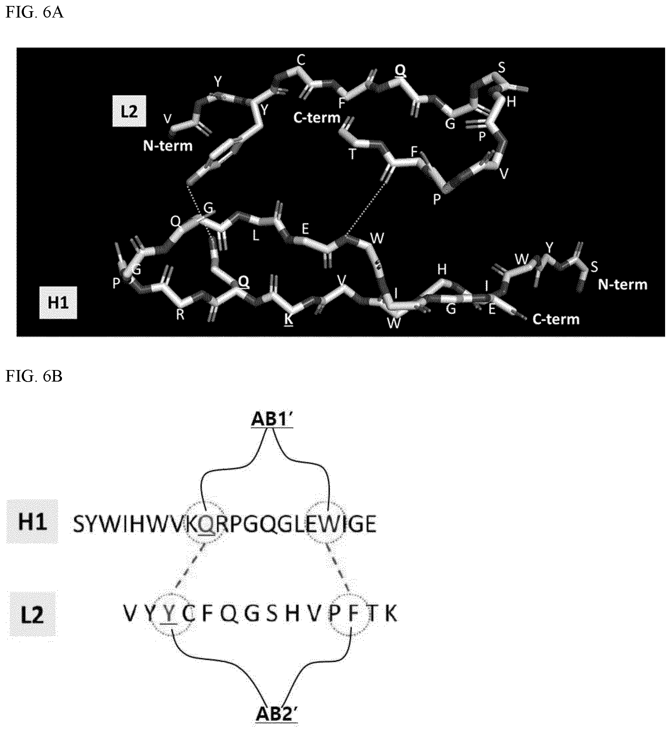

In an embodiment, the first switching binder (HA, HB) may include a first antibody-binding portion (AB1 in FIG. 5B and AB1' in FIG. 6B). By the first antibody-binding portion (AB1, AB1'), the first switching binder (HA, HB) can mainly bind to the framework region (FR') of the light chain (V_{L}) of the antibody 10. According to an embodiment, the first antibody-binding portion (AB1, AB1') may include at least one amino acid residue of - Y-, -I-, -W-, and -Q-. The first antibody-binding portion (AB1, AB1') may include an amino acid residue that specifically binds to the light chain (V_{L}) of the antibody 10 among the amino acid sequences selected from the heavy chain (V_{H}) of the antibody 10.

In an embodiment, preferably, the sequence comprising the first antibody-binding portion (AB1, AB1') may have the amino acid sequence of SEQ ID NO. 1 and SEQ ID NO. 2. The underlined amino acid residue in the amino acid sequence may be the first antibody-binding portion (AB1, AB1'). The amino acid sequence in the N-terminal to C-terminal direction of the amino acid sequence may be as follows.
SEQ ID NO. 1 (hereinafter referred to as H1): S-Y-W-I-H-W-V-K-Q-R-P-G-Q-G-L-E-W-I-G-E,
SEQ ID NO. 2 (hereinafter referred to as H2): V-Y-Y-C-A-R-E-P-T-G-T-G-I-Y-F-D-V-W-G-K.

In the case of SEQ ID NO. 1 (HI), the 9th amino acid residue Q and the 17th amino acid residue W in the amino acid sequence may be the first antibody-binding portion (AB1'). In the case of SEQ ID NO. 2 (H2), the 3rd amino acid residue Y, the 13th amino acid residue I, and the 18th amino acid residue W in the amino acid sequence may be the first antibody-binding portion (AB1). A detailed description thereof will be described in detail with reference to FIGS. 5A to 6E.

The above-mentioned SEQ ID NOs. 1 and 2 are only non-limiting embodiments, and the embodiment of the present disclosure may be any material comprising an amino acid sequence selected from the framework regions (FR) of the heavy chain (VH) capable of binding to each other.

In an embodiment, the second switching binder (LA, LB) may include a second antibody-binding portion (AB2 in FIG. 5B and AB2' in FIG. 6B). The second antibody-binding portion (AB2) allows the second switching binder (LA, LB) to bind to the framework regions (FR) of the heavy chain (V_{H}) of the antibody (10). According to an embodiment, the second antibody-binding portion (AB2, AB2') may include at least one amino acid residue of -Y-, -Q-, -P-, -L-, and -F-. The second antibody-binding portion (AB2, AB2') may include an amino acid residue that specifically binds to the heavy chain (V_{H}) of the antibody 10 among the amino acid sequences selected from the light chain (V_{L}) of the antibody 10.

In an embodiment, preferably, the switching binder 10 may include at least one of the amino acid sequences of SEQ ID NO. 3 and SEQ ID NO. 4, as the amino acid sequence comprising the second antibody-binding portion (AB2, AB2'). The underlined amino acid residue in the amino acid sequence may be the second antibody-binding portion (AB2, AB2'). The amino acid sequence in the N-terminal to C-terminal direction of the amino acid sequence may be as follows.
SEQ ID NO. 3 (hereinafter referred to as L1): T-Y-L-E-W-Y-P-Q-K-P-G-Q-S-P-K-L-L-I-Y-K,
SEQ ID NO. 4 (hereinafter referred to as L2): V-Y-Y-C-F-Q-G-S-H-V-P-F-T-K.

In the case of SEQ ID NO. 3, the 6th amino acid residue Y, the 8th amino acid residue Q, the 14th amino acid residue P, and the 16th amino acid residue L in the amino acid sequence may be the second antibody-binding portion (AB2). In the case of SEQ ID NO. 4, the 3rd amino acid residue Y and the 12th amino acid residue F in the amino acid sequence may be the second antibody-binding portion (AB2').

This is non-limiting and may include any amino acid sequence including an amino acid sequence selected from the antigen-binding portion 15 including the framework regions (FR) of the light chain (V_{L}) capable of self-binding to each other.

In an embodiment, the first antibody-binding portion (AB1, AB1') or the second antibody-binding portion (AB2, AB2') may be combined with the framework regions (FR, FR') of the heavy chain (V_{H}) or light chain (V_{L}) by at least one of electrostatic interaction, hydrogen bond, van der Waals force, hydrophobic interaction, and combinations thereof. For example, the first antibody-binding portion (AB1, AB1') and the second antibody-binding portion (AB2, AB2') constituting the switching binder 100 may be mutually bonded to the framework regions (FR, FR') by a hydrogen bond with a functional group of a side chain or a polar region of a peptide bond. However, the first antibody-binding portion (AB1, AB1') and the second antibody-binding portion (AB2, AB2') are not limited to mutual binding through the polar region of the peptide bond with the functional group by the side chain and may include various examples of binding capable of binding to the framework regions (FR, FR') of the switching binder 100 and the antibody 10, as in the case of polar as well as nonpolar bond. In another embodiment, the switching binder 100 may be designed to enable adjustment of binding strength, and a detailed description thereof will be described later with reference to FIG. 1B.

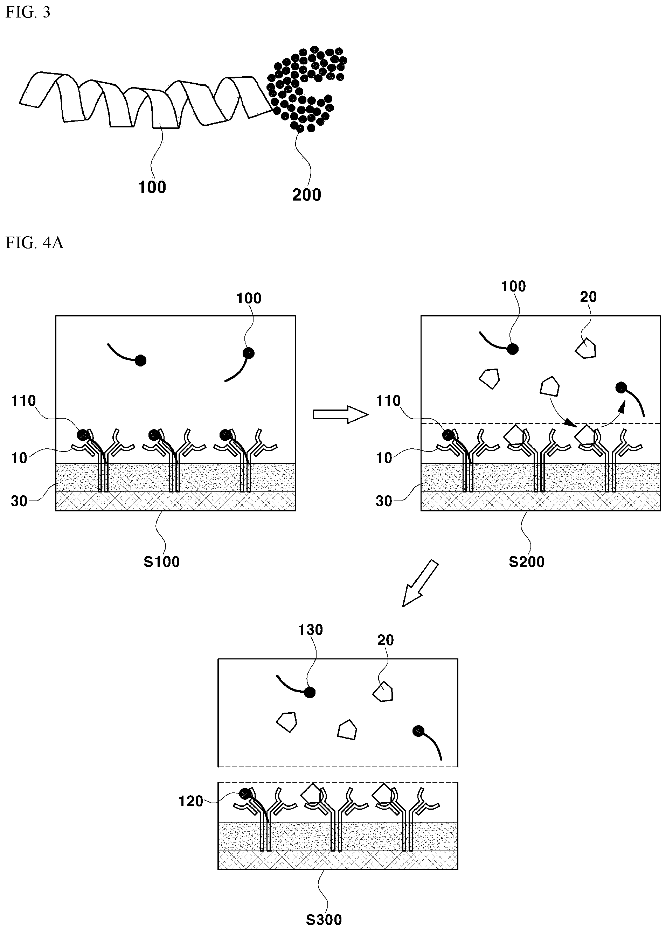

In an embodiment, the switching binder 100 reversibly bound to the framework regions (FR, FR') of the antibody 10 may be separated from the framework regions (FR, FR') when a target antigen (20 in FIG. 4A) that specifically reacts with the antibody 10 is bound to the antibody 10. When the target antigen 20 is combined with the antibody 10, the concentration of the antibody 10 in a state not bound to the target antigen 20 or the switching binder 100 may decrease. As the concentration decreases, the concentration of the antibody 10 to which the switching binder 100 is bound may also decrease in order to maintain the equilibrium of the binding reaction between the antibody 10 and the switching binder 100. In another embodiment, when the antibody 10 in which the switching binder 100 is bound to the framework regions (FR, FR') is bound to the target antigen 20, the switching binder 100 may be separated from the framework regions (FR, FR') by steric hindrance effect of the target antigen 20. This is a non-limiting example, and when the target antigen 20 binds to the antibody 10, the binding of the switching binder 100 and the framework regions (FR, FR') may be weakened or destroyed by various factors. The above-described examples do not limit the present disclosure.

In an embodiment, the switching binder 100 may not affect the binding process of the target antigen 20 to the antibody 10. For example, the switching binder 100 includes the amino acid sequence of a part of the framework regions (FR, FR') of an antibody, and binds to a part of the framework regions (FR, FR') or an adjacent region comprising thereof (HAR, HBR), and since the target antigen 20 binds to the antibody 10 by recognizing the complementarity determining regions (CDR, CDR') of the antibody 10, the target antigen 20 may bind to the antibody 10 to which the switching binder 100 is bound. Thereafter, the bond of the target antigen 20 and the antibody 10 may cause a steric hindrance effect on the target antigen 20, thereby causing separation of the switching binder 100 as bound to detect or analyze the target antigen 20.

In general, antigen-antibody reactions are difficult to control because many non-specific reactions occur, and a washing process or additional reagent treatment is required. The switching binder 100 of the present disclosure has a switching characteristic that performs a specific operation of binding and dissociation to the antibody 10 but enables antigen quantification and analysis in one step quickly and accurately while omitting the step of the washing process.

FIG. 1B is a diagram showing a basic configuration of a switching binder 100 according to an embodiment of the present disclosure.

Referring to FIG. 1B, the switching binder 100 may control the binding strength. For example, it is possible to control the binding strength of the switching binder 100 by amino acid modulation of the antibody-binding portion (AB). Specifically, it can be selected in consideration of the three-dimensional structure on the amino acid sequence of the framework regions (FR, FR') constituting the switching binder 100 and the antigen-binding portion 15 for mutual binding, and according to the modulation of the selected amino acid sequence, the binding strength of the antibody-binding portion 15 between the switching binder 100 and the framework regions (FR, FR') can be controlled. Through this, it is possible to provide the switching binder 100 capable of binding to various species of antibodies 10.

The switching binder 100 may include an antibody-binding portion (AB, referred to as the first antibody-binding portion (AB1, AB1') or the second antibody-binding portion (AB2, AB2') in FIG. 1B) and a binding strength regulating portion (BR) coupled to the antibody-binding portion (AB) as a basic unit configuration. FIG. 1B illustrates an 'antibody-binding portion (AB)-binding strength regulating portion (BR)' in which the antibody-binding portion (AB) and the binding strength regulating portion (BR) are connected to each other as a single unit of the basic configuration, but this is exemplary. For example, the antibody-binding portion (AB) and the binding strength regulating portion (BR) are included as a unit structure so that the antibody-binding portion (AB) and the binding strength regulating portion (BR) are continuously coupled to implement an expanded polymer chain, as -antibody-binding portion (AB)-binding strength regulating portion (BR)-antibody-binding portion (AB)-, -antibody-binding portion (AB)-binding strength regulating portion (BR)-antibody-binding portion (AB)-binding strength regulating portion (BR)-.

The antibody-binding portion (AB) may include an amino acid sequence of the switching binder 100 capable of recognizing and binding to various different species of antibodies. The antibody-binding portion (AB) may be selectively combined and then separated again specifically for at least a portion of the framework regions (FR, FR') of the variable region included in the antigen-binding portion of the heavy or light chain of an antibody having the same or similar amino acid sequence for various types of antibodies. That is, it can be reversibly coupled. These characteristics may occur, for example, depending on the structural characteristics of the switching binder 100 to be described later, but are not limited thereto. This includes the cases where all or part of the switching binder 100 reversibly binds to at least a part of the framework region of an antibody and since the antibody-binding portion 101 is bound to the framework region (FR) of the antibody, it is applicable to various antibodies.

In addition, the switching binder 100 features idiotope through which the antibody selectively binds to the antigen, i.e., it binds selectively to the framework region (FR) present between these complementarity determining regions (CDRs) rather than the complementarity determining region (CDR), and thus, unlike the anti-idiotype antibody used to interfere with binding to a specific antigen, it has the property of binding to various antibodies, particularly IgG antibodies, regardless of antigen-binding specificity.

Since the binding strength regulating portion (BR) can interfere with the binding of the antigen to the antibody as the binding strength between the switching binder 100 and the antibody 10 is stronger, in order not to affect the antigen-antibody binding coefficient for immunoassay, the binding strength between the switching binder 100 and the antibody can be adjusted. In an embodiment, the binding strength regulating portion (BR) may have a sequence of amino acids that are distinguished from each other according to the types of antibodies to which they are applied. The binding strength of the switching binder 100 bound to each antibody may be regulated by the amino acid sequence difference of the binding strength regulating portion (BR). Accordingly, the binding strength of the switching binder 100 bound to the antibody may vary depending on the type of antibody in the antigen-antibody reaction. Since the binding strength between the antibody-binding portion (AB) of the switching binder 100 and the antibody can be controlled by the binding strength regulating portion (BR), the antibody-binding portion (AB) can be designed to be easily separated from the framework region (FR) of the antibody 10 under certain conditions in response to various types of the antibody 10 so that the switching binder 100 can be separated from the antibody 10. For example, when the antibody-binding portion (AB) is bound to the framework region (FR) of the antibody 10, and the target antigen recognizes and specifically binds to the antigen-binding portion of the antibody, it enables the antibody-binding portion (AB) to be easily separated from the framework region (FR) of the antibody 10 and the target antigen to be easily bound to the antibody. Accordingly, the switching binder 100 having a unit structure of the antibody-binding portion (AB)-binding strength regulating portion (BR) according to an embodiment of the present disclosure has a switching characteristic that performs a specific action of binding and dissociation to the antibody in the antibody-antigen reaction.

As described above, the switching binder 100 of the present disclosure is bound to a predetermined antibody 10, and then when the target antigen 20 is bound to the antibody, the switching binder 10 is separated from the antibody 10 and detects the isolated switching binder, which thereby makes it possible to specify and quantify the antigen 20. A known method may be used for the identification and quantification of the antigen 20. That is, the switching binder 100 binds to the antigen-binding portion of the antibody 10 and is separated only when the antigen is bound to the antibody. Although the antibody-binding portion (AB) of the switching binder 100 according to an embodiment of the present disclosure binds rather non-specifically to a wide range of various antibodies, when the target antigen 20 is bound to the antibody 10 by the binding strength regulating portion (BR), it allows for the entire switching binder 100 to be easily separated from the antibody 10, and for the switching binder 100 to cause a specific reaction to the target antigen 20, that is, a separation reaction from the antibody 10, and thereby the antigen can be titrated and/or quantified using the isolated switching binder. In addition, unlike the anti-idiotype antibody used to interfere with binding to a specific antigen, it may have non-limiting properties of binding to an antibody regardless of antigen-binding specificity. Furthermore, by using these properties of the switching binder 100, applications to not only diagnostic agents but also therapeutic agents and biosensors can be derived. As described above, so as not to affect the binding of a specific antigen to the antibody 10, the binding strength of the switching binder 100 to the antibody 10 may be designed to be smaller than the binding strength between the antibody 10 and a specific antigen. However, the present disclosure is not limited thereto. In another embodiment, the binding strength of the switching binder 100 to the antibody 10 greater than the binding strength of the antibody 10 and the target antigen 20 can control the binding reaction between the antigen and the antibody 10. This can be applied when it is necessary to control the intensity of a reaction between the antigen and the antibody in the diagnostic field. The antibody-binding portion (AB) is a site capable of controlling binding strength by modulation, and a detailed and specific description thereof will be described later with reference to FIGS. 5A to 6E.

In addition, the binding strength regulating portion (BR) can be bound to and separated from a variety of antibodies rather than a specific antibody. Even when the binding strength regulating portion (BR) binds to an antibody of an unknown antigen such as a mutant virus, the binding strength of the switching binder 100 can be regulated to facilitate separation of the switching binder 100 from the antibody 10, which can improve the rapidity and accuracy of the identification and quantification of antibodies to be described later. Embodiments of the present disclosure have the advantage of enabling rapid and accurate one-step quantification and analysis of antigens while omitting the step of the washing process by using the switching operation of binding and dissociation of the switching binder 100 in the conventional antigen-antibody reaction which requires the washing process due to many non-specific reactions and is difficult to control.

In an embodiment, the antibody-binding portion (AB) may refer to the above description for the first antibody-binding portion (AB1, AB1') and the second antibody-binding portion (AB2, AB2') in FIG. 1A. In other embodiment, the binding strength regulating portion (BR) may comprise at least one of the amino acid residues or sequences such as the amino acid sequences -S-Y-W-I-H-W-V-K-, -R-P-G-Q-G-L-E-, -I-G-E-, -V-Y-, - C-A-R-E-P-T-G-T-G-, -Y-F-D-V-, -G-K-, -T-Y-L-E-W-Y-, -K-P-G-Q-S-, -K-, L-I-Y-K-, -C-F-Q-G-S-H-V-P- and -T-K- (hereinafter, binding strength regulating portion amino acid sequence), but is not limited thereto. As described above, the switching binder 100 includes the antibody-binding portion (AB) and the binding strength regulating portion (BR) as one or more unit structures, such that it can be expressed by the binding of extended sequences in which the antibody binding part (AB) and the binding strength regulating portion (BR) are continuously linked. Accordingly, the binding strength regulating portion (BR) may modulate the position of the antibody-binding portion (AB) in the switching binder 100 to control the binding strength of the antibody-binding portion (AB) to the antibody 10. In an embodiment, the switching binder 100 may include an amino acid sequence in which an antibody-binding portion (AB) selected from any one of the antibody-binding portion (AB) amino acid sequences and a binding strength regulating portion 102 selected from any one of the binding strength regulating portion (BR) amino acid sequences are alternately repeated at least once or more. For example, it is any sequence that is physically and chemically possible such as -T-Y-L-E-W-Q-K-W-, -V-Y-Y-, -V-Y-Y-G-K-, -K-P-G-Q-S-I-, -G-K-W-K-L-, -Y-F-D-V-W-G-K-, -F-C-F-Q-G-S-H-V-P-Q-, -R-P-G-Q-G-L-E-F-V-Y-, -S-Y-W-I-H-W-V-K-Q-T-K-F-, and W-I-G-E-Y-V-Y-Q-Y-F-D-V-P-. It may include, but is not limited to, those in which the antibody-binding portion amino acid sequence and the binding strength regulating portion amino acid sequence are alternately repeated at least once or more. In other embodiment, the number of consecutively bound and extended amino acid residues by including the antibody-binding portion (AB) and the binding strength regulating portion (BR) as one unit structure in the switching binder 100 may be the same or more compared to the number of amino acid residues of the framework regions (shown in FIG. 2, FR) of the binding antibody, but not limited thereto, and thus may include fewer cases. For example, the number of the extended amino acid sequence is 6 to 30, but is not limited thereto.

FIG. 2 is a diagram illustrating the binding of a switching binder 100 to a framework region (FR) and a complementarity determining region (CDR) adjacent to the framework region (FR) according to another embodiment of the present disclosure.

Referring to FIG. 2, according to an embodiment of the present disclosure, the switching binders 100a and 100b may be bound to a combination of all or part of the FR1 region (FR1) in the framework regions (FR), all or part of the FR2 region (FR2) in the framework regions (FR), all or part of the FR3 region (FR3) in the framework region (FR), or all or part of the R4 region (FR4) in the framework regions (FR). For example, the switching binder 100a may be bound to the FR1 region (FR1) of the framework regions (FR), and the switching binder 100b may be bound to the FR2 region (FR2). When the switching binder 100 is bound to a portion of the framework regions, it may be bound to the framework regions over about 30% to 70% thereof. However, the switching binders 100a and 100b shown in FIG. 2 are bound only to the FR1 region (FR1) of the framework region (FR) and only to the FR2 region (FR2) of the framework region (FR), but are not limited thereto. They can also bind to the FR3 region (FR3) of the framework region or the FR4 region (FR4) of the framework region. As described above, they can also bind to a sequence used by linking the framework regions (FR) such as the FR1 region (FR1) of the framework region (FR) to the FR2 region (FR2) of the framework region (FR) or the FR2 region (FR2) of the framework region (FR) to the FR4 region (FR4) of the framework region (FR).

According to an embodiment of the present disclosure, the switching binder 100c may simultaneously bind to at least one framework region (FR) and at least one complementarity determining region (CDR). For example, a portion of the entire amino acid sequence of the switching binder 100c may be bound to a framework region (FR), and another portion may be bound to a complementarity determining region (CDR). In other embodiment, the complementarity determining region (CDR) may be a region extending from the framework region (FR) to which the switching binder 100c is bound. For example, when a portion of the amino acid sequence of the switching binder 100c binds to the FR2 region (FR2), the other portion may be bound to the CDR1 region (CDR1) of the complementarity determining region (CDR) or the CDR2 region (CDR2) of the complementarity determining region (CDR). However, this is non-limiting and may be similarly bound to the complementarity determining region (CDR) located between the framework regions (FR). For example, the switching binder 100 can be consecutively bound to the CDR4 region (CDR4) of the complementarity determining region (CDR) between the FR3 region (FR3) in the framework region (FR) and the FR4 region (FR4) in the framework region (FR).

Referring to FIG. 2, although disclosed with respect to switching binders bound to the heavy chain (V_{H}), the switching binders according to an embodiment of the present disclosure can be similarly bound to the framework region (FR') of the light chain (V_{L}).

In an embodiment, the antibody 10 may include any antibody, unless otherwise specified, including an isotype, hollotype, or idotype antibody. In particular, when the antibody 10 is an isotype antibody, the antibody 10 may include an antibody such as IgG, IgA, IgM, IgD, or IgE. According to another embodiment, the antibody 10 may be a human antibody, a non-human antibody, a humanized non-human antibody, or a combination thereof. As described above, the switching binder 100 comprises framework regions (FR, FR') constituting the antigen-binding portion 15 that causes mutual binding to form a three-dimensional structure of the antibody, and an amino acid sequence selected from complementarity determining regions (CDR, CDR') adjacent to framework regions (FR, FR'). The amino acid sequence of the framework regions (FR, FR') may be maintained similarly to the complementarity determining region (CDR, CDR') with little change even if the type of antibody 10 changes. Therefore, it is possible to non-specifically bind to various antibodies 10 by having the same or substantially similar amino acid sequence to the source animal of the antibody 10, for example, mouse, rabbit, goat, or human. Accordingly, the switching binder 100 can be applied to various antibodies and have an extended usable range.

According to an embodiment, the switching binder 100 may be a peptide, a protein, an aptamer, a nanobody, a phage display, a yeast display, or another antibody.

The peptide can be produced by chemical synthesis, solid-phase technology using amino acid precursors, and synthetic or recombinant means by an automated peptide synthesizer. The above-described methods for synthesizing peptides are non-limiting examples, and various known methods for synthesizing peptides may be applied.

The protein is a high molecular compound composed of several amino acids formed by peptide bonds of amino acids. In an embodiment, the protein can be synthesized using automated organic synthesis methods, and a genetically engineered recombinant protein can be expressed such that a nucleic acid sequence encoding a macromolecular delivery domain is cloned into a nucleic acid expression vector.

The aptamer may be obtained by an in vitro selection method using the binding of a target molecule as a non-limiting example. Selection of aptamers that specifically bind to the framework regions (FR, RR') of the target molecule, in particular, the heavy chain (V_{H}) or light chain (V_{L}) can be achieved by the separation of the aptamers that can specifically bind to each ligand from organic molecules, nucleotides, amino acids, peptides, polypeptides, marker molecules on the cell surface, ions, metals, salts, and polysaccharides. In addition, the selection of the aptamers may be performed using an in vivo or in vitro selection technique using the systematic evolution of ligands by exponential enrichment (SELEX) method.

Nanobody is a single domain antibody and a monomer variable antibody domain composed of a single antibody fragment and can selectively bind to a specific antigen like an antibody. Nanobodies may comprise heavy chain antibodies, antibodies naturally devoid of light chains, single domain antibodies derived from conventional four-chain antibodies, engineered antibodies, and single domain scaffolds other than those derived from antibodies.

In addition, phage display is to present a part or all of a protein such as a peptide or an antibody on the surface of a bacteriophage. It is widely used for the identification of protein-protein interactions through the phage display, and in addition, it is useful for the search for important specific antibodies for treatment, diagnosis, or experimentation. Yeast display is a protein engineering technique that uses the expression of recombinant proteins integrated into the yeast cell wall to isolate and engineer antibodies.

The antibody as the switching binder 100 may be a human antibody, a non-human antibody, or a humanized non-human antibody. The human antibody may be an antibody having an amino acid sequence that corresponds to an antibody produced by a human and/or has been prepared using any technique for making a human antibody as disclosed herein. The human antibody may be formed by a phage-display or human hybridoma technique. The non-human antibody may be an antibody obtained from a source of various species, for example, non-human mammals or birds except humans such as rodents, rabbits, cattle, sheep, pigs, and dogs. The humanized non-human antibody is a chimeric antibody that contains minimal sequence derived from non-human immunoglobulin. In an embodiment, the humanized non-human antibody may also contain a sequence selected from non-human antibodies instead of a human sequence. In another embodiment, the humanized antibody may contain conservative amino acid substitutions, i.e., non-natural residues derived from the same species or other species that do not significantly alter antibody binding and/or biological activity. These modifications can be made to further refine antibody performance, such as binding affinity. Examples of the above-described switching binder 100 are non-limiting examples and may include various examples including the above-described amino acid sequence capable of reversibly binding to a heavy or light chain of an antibody.

According to an embodiment, even if a part of the amino acid sequence in the switching binder 100 is replaced with an inorganic material, it may bind to the antigen-binding portion 15 including the framework regions (FR, FR') of the heavy or light chain of the antibody 10, and the switching binder 100 may include a corresponding substance. The inorganic material may be, for example, selected from the group consisting of silicon dioxide (SiO₂), aluminum oxide (Al₂O₃), calcium oxide (CaO), magnesium oxide (MgO), iron oxide (Fe₂O₃), titanium oxide (TiO₂), potassium oxide (K₂O), sodium oxide (Na₂O), limestone (CaCO₃), dolomite (CaMg(CO₃)₂), talc (Mg₃Si₄O₁₀(OH)₂), silver oxide (Ag₂O), chronium oxide (Cr₂O₃), cobalt oxide (CoO₂), copper oxide (CuO), and zinc oxide (ZnO).

FIG. 3 shows a switching binder 100 according to an embodiment of the present disclosure.

Referring to FIG. 3, according to an embodiment, the switching binder 100 may be labeled with a labeling material 200 that exhibits a chemical, electrical, magnetic, or optical labeling reaction. In the antigen-antibody reaction with the label of the labeling material 200, the switching binder 100 has the advantage of being able to identify and quantify an antibody and an antigen by the amount of label shown by binding and separating the antibody 10, and to measure the binding strength of the antigen to the antibody, which will be described later.

In the case of the chemical labeling reaction, an oligohistidine sequence-tag, chemical tagging with a dye, or the like may be included. In the case of the electrical labeling reaction, an electron current signal reaction generated by an oxidation-reduction reaction of an expression substrate may be included. In the case of the magnetic labeling reaction, a specific radiolabeled binding member, specifically an antibody molecule may be loaded with a plurality of paramagnetic ions via a chelating group. The chelating groups may include EDTA, porphyrin, polyamine crown ethers, and polyoximes. Examples of the paramagnetic ions may include gadolinium, iron, manganese, rhenium, europium, lanthanum, holmium, and erbium. The paramagnetic ions are loaded to allow identification and localization of the antibody via radiolabeled specific binding members, particularly antibodies and fragments of antibodies. In the case of the optical labeling reaction, luminescence, color development, fluorescence, turbidity, magnetic force, or electrical resistance reaction may be included.

In an embodiment, the labeling material 200 may be bound to at least any part of the switching binder 100. The labeling material 200 may be a chromogenic material, a light emitting material, a fluorescent material, a magnetic material, a metal material, an organic synthetic material, or a combination thereof. As an embodiment of the chromogenic material, the labeling material 200 may be horseradish peroxidase (HRP), and the substrate may be a peroxide such as hydrogen peroxide and/or 3,3',5,5'-tetramethylbenzidine (TMB). Alternatively, the labeling material 200 may be alkaline phosphatase (AP), and the substrate may be a material such as bromochloroindolyl phosphate (BCIP), nitro blue tetrazolium (NBT), naphthol-ASB1-phosphate, and enhanced chemifluorescence (ECF). The labeling material 200 may act as an enzyme that decomposes the substrate to exhibit an optical reaction, a chemical reaction, or an electrical reaction. Examples of the fluorescent material may include HEX^{™}, TAMRA (Applied Biosystems), Cy5 (Lumiprobe), or sulforodamine 101 acid chloride (Texas Red), preferably FAM fluorescence or TAMRA fluorescence. Examples of the metal material may include any coordinated metal atom capable of undergoing an oxidation-reduction reaction, for example, a material such as iron, cobalt, ruthenium, zinc, copper, lithium, or silver. Some specific examples may be selected from the group consisting of ferrocene, zinc tetrabenzoporphyrin, cobalt phthalocyanine, tris-(2,2'-bipyrimidine) ruthenium, 4-ferrocenylbenzyl alcohol, 5-(4-hydroxymethylphenyl)-10,15,20-trimesityl porfinatozinc (II), and redox-active calixarene. Examples of organic synthetic material may include fluorescein isothiocyanate, rhodamine, phycoerythrin, phycocyanin, allophycocyanin, or fluorecamine. This is a non-limiting example, and various types of known techniques for labeling the switching binder 100 may be referred to.

Referring back to FIGS. 1A to 3, in an embodiment, a method for preparing a switching binder 100 may include the steps of identifying an amino acid sequence at an antigen-binding portion 15; obtaining a hydrophobicity index of the amino acid sequence; comparing the hydrophobicity index to extract the switching binder 100. The description of the switching binder 100 is the same as that described above without contradiction.

In the step of identifying the amino acid sequence, an amino acid sequence having the characteristics of self-binding between the antigen-binding portions 15 can be identified in the antigen-binding portion 15 including the framework regions of the heavy and light chains of the antibody 10. Compared to the complementarity determining regions (CDR, CDR'), the framework regions (FR, FR') have the same or substantially similar amino acid sequence between various types of antibodies and are applicable to various antibodies, such that the amino acid sequence to be selected in the framework regions (FR, FR') can be identified.

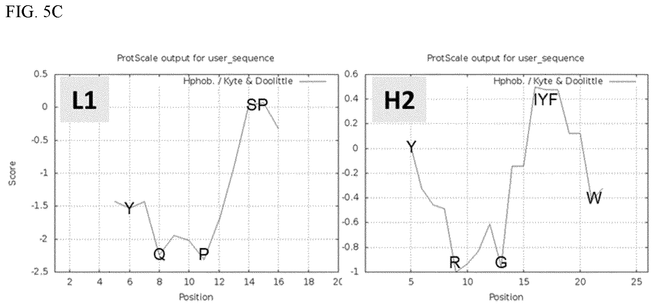

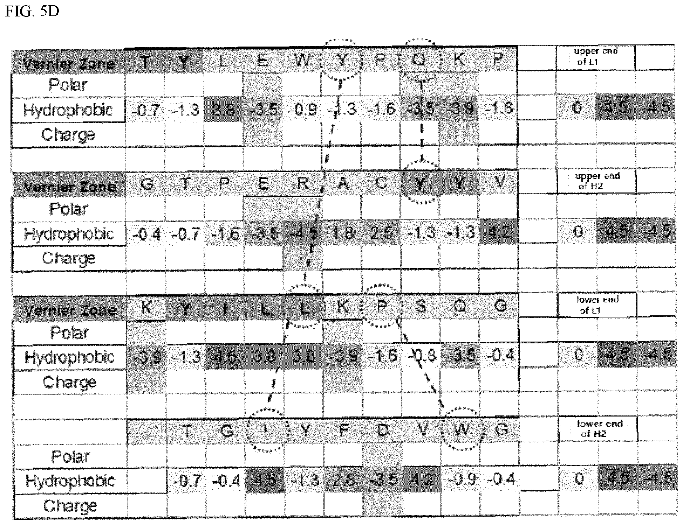

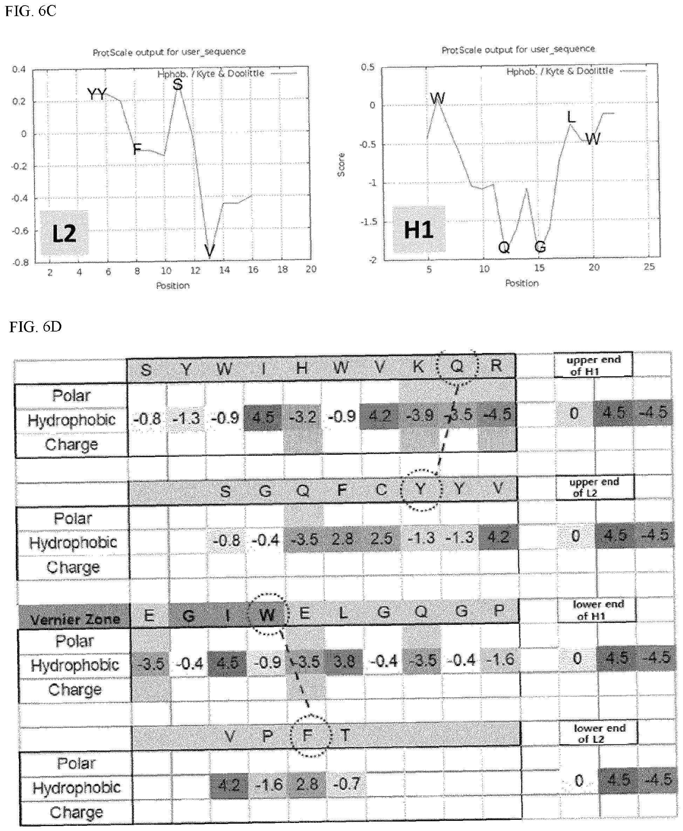

In the step of obtaining a hydrophobicity index of the amino acid sequence, the hydrophobicity index for each amino acid sequence site for the antibody can be obtained. The hydrophobicity index can be divided into negative and positive values. It can be confirmed that the positive value indicates a hydrophilic property, and the negative value indicates a portion having a hydrophobic property. A detailed description thereof will be described later with reference to FIGS. 5C and 5D and FIGS. 6C and 6D.

It may include the step of extracting the switching binder 100 comprising the amino acid sequence of a portion having the same properties between the amino acid sequences of the framework regions (FR, FR') of the heavy chain (V_{H}) and light chain (V_{L}) constituting the antigen-binding portion 15 by comparing the hydrophobicity index. In an embodiment, inter-sequences exhibiting hydrophilic properties or sequences exhibiting hydrophobic properties may be mutually bound. Through this, the amino acid sequence of a portion capable of mutual binding due to hydrophilic properties or a portion having hydrophobic properties capable of mutual binding may be selected as all or part of the amino acid sequence of the switching binder 100. Specifically, in the step of selecting the switching binder 100, through the information of the amino acid sequence, the amino acid sequence having mutual binding properties to the framework regions (FR, FR') of the heavy chain (VH) or light chain (VL) is extracted. Through the property of binding between the antigen-binding portions 15 including the framework regions (FR, FR') of the antibody 10, binding of the switching binder 100 to the framework regions (FR, FR') can have the same effect as the interaction between the framework regions (FR, FR'). A detailed description of the switching binder 100 is the same as described above.

In another embodiment, as a pharmaceutical composition for disease screening, diagnosis, prophylaxis, treatment, or integration of diagnosis and treatment, the switching binder 100 may be included. The switching binder 100 has the advantage of enabling diagnosis, prevention, or treatment of various viruses through the characteristic that it specifically binds and isolates the framework regions of various antibodies 10. The pharmaceutical composition may be administered in various oral and parenteral formulations at the time of administration. In the case of formulation, it can be prepared using a diluent or excipient such as a filler, extender, binder, wetting agent, disintegrant, or surfactant, commonly used.

Solid formulations for oral administration include tablets, pills, powders, granules, capsules, etc., and such a solid formulation is prepared by mixing one or more compounds with at least an excipient, for example, starch, calcium carbonate, sucrose or lactose, gelatin, and the like. In addition to simple excipients, lubricants such as magnesium stearate and talc are also used. Liquid formulations for oral administration include suspensions, internal solutions, emulsions, syrups, etc. In addition to water and liquid paraffin which are commonly used as simple diluents, various excipients such as wetting agents, sweeteners, fragrances, preservatives, etc. may be included. For a description of the switching binder 100, a reference may be made to the foregoing.

Formulations for parenteral administration may include sterile aqueous solutions, non-aqueous solutions, suspensions, and emulsions. As the non-aqueous solvent and the suspending agent, propylene glycol, polyethylene glycol, vegetable oils such as olive oil, and injectable esters such as ethyl oleate may be used.

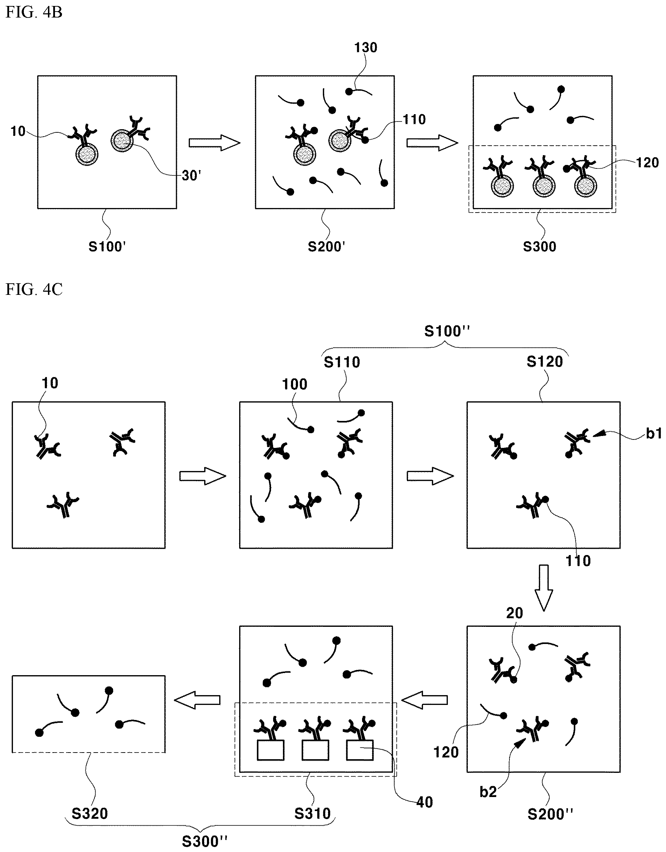

FIG. 4A is a flow diagram of an assay method using a switching binder 100 according to an embodiment of the present disclosure, and FIG. 4B is a flow diagram of an assay method using a switching binder 100 according to another embodiment of the present disclosure, and FIG. 4C is a flow diagram of an assay method using a switching binder 100 according to still another embodiment of the present disclosure.

Referring to FIG. 4A, the assay method according to an embodiment may include the step of selecting an amino acid sequence from the framework regions (FR, FR') (not shown). The amino acid sequence of the switching binder 100 can be selected from the framework regions (FR, FR') in which the heavy chain (V_{H}) and light chain (V_{L}) of the antibody are located adjacent to each other and self-associate with each other. Regarding the step of selecting the amino acid sequence, the disclosure of FIGS. 1A to 2 and the following description may be referred to as those that can be selected from the framework regions (FR, FR') constituting the antigen-binding portion 15 of the antibody 10. Therefore, there is an advantage that can be applied to various antibodies 10 by extracting the amino acid sequence including the same or substantially similar framework regions (FR) for each antibody 10 among the antigen-binding portions 15 of the antibody 10.

In an embodiment, the antibody 10 may be prepared by immobilizing to the support 30. The support 30 may be a substrate, a bead, a fiber, or a polymer structure. A substrate that can be used as the support 30 is not limited as long as it can be used for a biochip or a biosensor. Preferably, it may be gold, ceramics, glass, polymers, silicon, modified silicon, fibers, and metals whose surface has been modified or unmodified by a separate treatment. Specifically, the polymer may include tetrafluoroethylene, polystyrene, polycarbonate, or polypropylene. The fiber may include nitrocellulose, regenerated cellulose, or nylon membrane. Materials that can be treated on the substrate to modify the surface may be materials such as polymers, plastics, resins, carbohydrates, silica, silica derivatives, carbon, metallic inorganic glass, and films. The support 30 serves not only as the support 30, but also provides a place for the reaction between the immobilized antibody and the sample. The specification of the support and the position, size, and shape of the antibody fixed thereon may vary depending on the purpose of analysis, a spotting machine, a dispensing machine, and an analysis device such as a scanner.

FIG. 4A shows that the antibody 10 is immobilized on a substrate using a solid support 30, but is not limited thereto, and the antibody 10 is fixed to a bead and placed in a solution (see FIG. 4B); the antibody 10 is not immobilized, but may be provided in a mixed state in a solution (see FIG. 4C ). That is, it can be applied to immunosorbent-type immunoassays and homogeneous-type immunoassays.

In an embodiment, the switching binder 110 bound to the antibody 10 may be quantified in the pretreatment step (SI00). For example, antibody 10 is immobilized on a substrate, and the switching binder 100 shows a labeling reaction by binding the labeling material 200 to the switching binder 100. The intensity of the labeling reaction of the switching binder 100 on the lower surface of the substrate may be measured. According to an embodiment of the present disclosure, before the target antigen 20 is bound to the antibody 10, the high-accuracy identification or quantification of the target antigen 20 can be achieved by quantifying the switching binder 110 bound to the antibody 10 and using the amount of the switching binder 110 bound to the antibody 10 as an initial value. For a detailed description of the switching binder 100, the labeling material 200, and the labeling reaction, a reference may be made to the foregoing disclosure. In addition, as used herein, the "amount" of the switching binder 100 may be used interchangeably with the "concentration" of the switching binder 100. A description that the amount of the switching binder 100 is "high" or "small" may be understood as "high" or "low" the concentration of the switching binder 100.

Then, the step of providing the analyte to the antibody 10 may be included. Specifically, the antigen processing step (S200) of binding the target antigen 20 to the antibody 10 and separating the bound switching binder 110 from the framework regions (FR, FR') may be performed. The target antigen 20 may or may not be included in the analyte. When the target antigen 20 is included in the analyte and the target antigen 20 is provided to the antibody 10, at least some of the switching binder 110 bound to the antibody 10 may be separated from the framework regions (FR, FR') and released into a solution phase containing the antibody 10 by the specific reactivity of the target antigen (20) with the antibody (10). As the concentration of the target antigen 20 increases, the ratio of the switching binder 100 separated from the antibody 10 among the switching binder 110 bound to the antibody 10 may increase.

After the antigen processing step, the assay step of quantifying at least one of the remaining amount of the switching binder 120 bound to the framework regions (FR, FR') or the switching binder 130 separated from the framework regions (FR, FR') and analyzing the target antigen 20 may be performed. According to an embodiment of the present disclosure, the target antigen 20 in the analyte can be analyzed through the switching binders 100, 120, and 130 without performing a washing step after providing the analyte to the antibody 10. Rapid analysis is possible and no additional equipment is required for washing. In addition, since the step of labeling the antibody itself is not included, one-step simplification is possible. Even if a small amount is used, the antigen-antibody reaction due to antigen binding can be identified with a material labeled with the switching binder 100, and thus miniaturization is possible. Therefore, the present disclosure can provide a one-step immunoassay with improved ease of use.

In an embodiment, a residual amount of the switching binder 120 bound to the framework regions (FR and FR') may be quantified. In this case, if the amount of the switching binder 120 of the residual amount bound to the framework regions (FR, FR') measured in the assay step is reduced than the amount of the switching binder 110 bound to the antibody 10 in the pretreatment step (S100), information regarding whether the target antigen 20 is included in the analyte may be acquired. This is because the switching binder 110 is separated only when the target antigen 20 is bound to the antibody 10.

In addition, the greater the difference between the residual amount of the switching binder 120 bound to the framework regions (FR, FR') measured in the assay step and the amount of the switching binder 110 bound to the antibody 10 in the pretreatment step (S100), information indicating that the amount of the target antigen 20 is large or information indicating that the concentration is high may be obtained. Accordingly, the present disclosure can identify or quantify up to the amount or concentration of the target antigen 20 contained in the analyte (hereinafter referred to as the first assay).

In other embodiment, the switching binder 130 separated from the framework regions (FR and FR') may be quantified. In this case, when the amount of the switching binder 130 separated from the framework regions (FR, FR') is equal to or greater than the threshold value, the target antigen 20 may be included in the analyte (hereinafter referred to as the second assay).

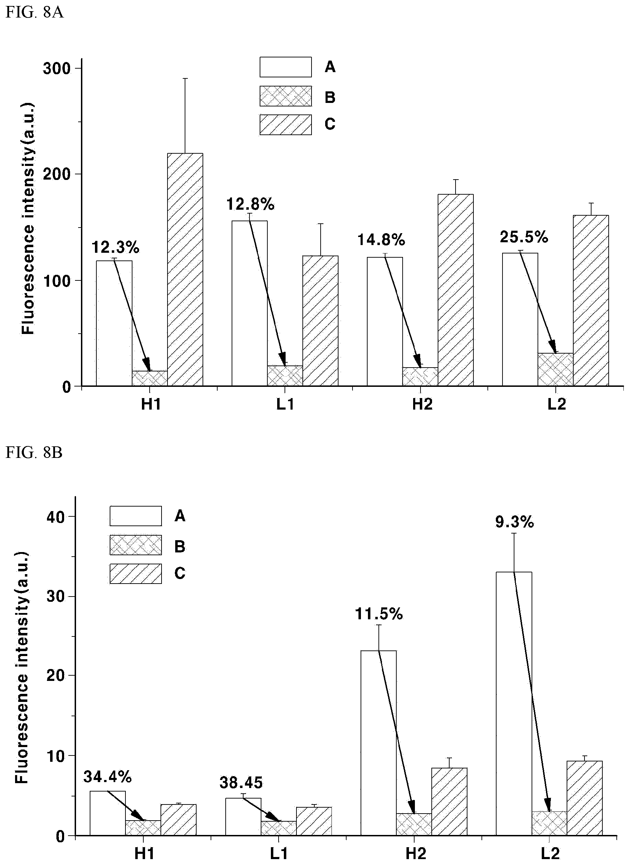

In another embodiment, both the residual amount of the switching binder 120 bound to the framework regions (FR, FR') and the switching binder 130 separated from the framework regions (FR, FR') may be quantified. For a detailed description of the quantitative result assay (the first assay and the second assay), a reference may be made to the foregoing disclosures. Accordingly, two assay methods may be utilized for quantification of the target antigen 20 using the difference between the amount of the switching binder 110 bound to the antibody 10 measured in the pretreatment step (S100) and the remaining amount of the switching binder 120 bound to the framework regions (FR, FR') in the assay step (S300) (the first assay) and the difference in the amount of the switching binder 130 separated from the framework (FR, FR') (the second assay). Through the first assay and the second assay, it is possible to provide a one-step immunoassay with improved accuracy and reliability of the quantitative analysis of the target antigen 20. The quantification can be determined by measuring the above-described labeling reaction of the labeling material 200 bound to the remaining amount of the switching binder 120 and/or the separated switching binder 130. For example, the amount of fluorescence in the labeling reaction may be measured using a fluorescence resonance energy transfer (FRET) method. Through this, the switching binder 100 is released according to the quantitative relationship to the concentration of the antigen 20 from the antibody 10 when binding the antigen 20 at different concentrations. Accordingly, it is possible to implement a one-step immunoassay capable of quantifying the analyte, for example, the antigen (20). Assay results for the assay method disclosed in FIG. 4A will be described in detail with reference to FIGS. 8A to 8D.

Referring to FIG. 4B, the assay method according to an embodiment may include the step of selecting an amino acid sequence from the framework regions (FR, FR') (not shown). The description of step of selecting the amino acid will be described in detail with reference to FIGS. 1A to 2 and the embodiments to be described later.

In an embodiment, the antibody 10 may be immobilized on the beads in the pretreatment step (SI00'). The beads are non-limiting examples and may be glass beads, magnetic beads, or polymer beads. According to an embodiment of the present disclosure, as the support 30 for immobilizing the antibody 10, beads that can be disposed of or dispersed in a solution rather than a substrate are used. Thus, it is easy to uniformly immobilize the antibody 10 to the beads, and afterward, in the antigen processing step (S200'), the antibody 10 and the target antigen 20 are mixed, so that the degree of binding between the antibody 10 and the target antigen 20 increases. In addition, the beads may be dispersed or disposed of in a solution, unlike fixed support such as a substrate, and a solution-phase process for analysis of the target antigen 20 is possible. As a result, the step of fixing the antibody 10 can be omitted, and the target antigen 20 and the antibody 10 bind with a high probability, thereby improving the accuracy of the assay.

Thereafter, as described in FIG. 4A, in the step of providing the analyte, specifically, the antigen processing step (S200'), the step of separating the switching binder 100 from the framework regions (FR, FR') by binding the target antigen 20 as an analyte to the antibody 10 present in the solution may be performed. For a detailed description of the separation of the switching binder 100 from the framework regions (FR, FR'), a reference may be made to the foregoing description.

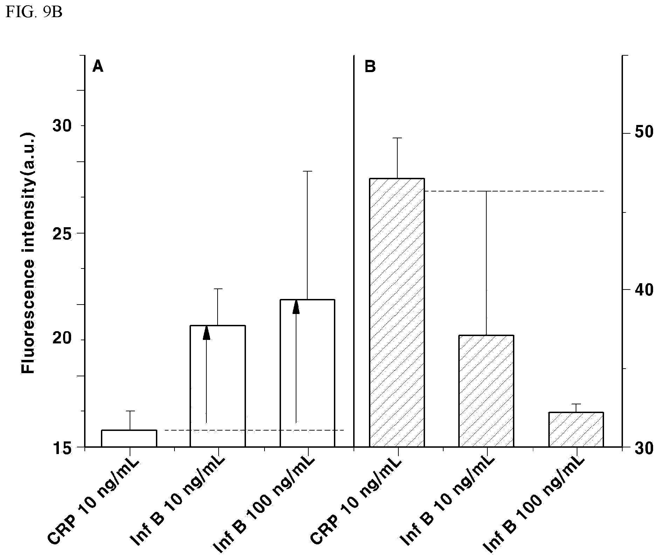

Then, in the assay step (S300'), after the antigen processing step, the remaining amount of the switching binder 120 bound to the framework regions (FR, FR') and the switching binder 130 separated from the framework regions (FR, FR') may be separated. For example, the switching binder of the remaining amount of the switching binder 120 bound to beads, an antibody 10 immobilized on a bead, and the framework regions (FR, FR') of the antibody 10 and the switching binder 130 separated from the framework regions (FR, FR') may be separated. The density of the switching binder may be greater than the density of the separated switching binder 130. In this case, the switching binder may be separated because the density of the switching binder is greater than the density of the separated switching binder 130. For this purpose, a centrifuge may be used, or the switching binder may be precipitated. Assay results for the assay method of FIG. 4B will be described in detail with reference to FIGS. 9B and 9C.

Referring to FIG. 4C, the assay method according to an embodiment may include the step of selecting an amino acid sequence from the framework regions (FR, FR') (not shown). For a description of the step of selecting the amino acid, a reference may be made to the details described in FIGS. 1A to 2.

The pretreatment step (S100") may include providing the switching binder 100 to an antibody 10 to obtain a mixed solution containing a first conjugate (b1) of the antibody 10 and the switching binder 100, and the switching binder 100 not bound to the antibody 10 (S110); and removing the switching binder 100 not bound to the antibody 10 from the mixed solution (S120). For example, the mixed solution passes through a desalting column to remove the unbound switching binder passing through the desalting column first, and only the first conjugate (b1) passing through the desalting column later can be obtained. The above-described method is exemplary, and various known techniques for separating the first conjugate and the switching binder 100 may be referred to.

Then, as the step of providing an analyte, in the antigen processing step (S200"), the step of separating the first conjugate (b1) from the framework regions (FR, FR') by binding the target antigen 20 to the antibody 10 may be performed. When the target antigen 20 is provided to the antibody 10, by the specific reactivity of the target antigen 20 with the antibody 10, the switching binder 100 bound to the antibody 10 in the first conjugate (b1) is separated into the framework regions (FR, FR') and released into a solution phase.

Then, the assay step (S300") may further include the steps of separating the second conjugate (b2) of the antibody 10 to which the switching binder 130 separated from the antibody 10 and the target antigen 20 are bound (S310); and quantifying the separated switching binder 130 (S320). The second conjugate (b2) and the separated switching binder 130 may be separated using, for example, a protein-A column 40. Thereafter, only the separated switching binder 130 may be selectively obtained and quantified through a labeling reaction. At this time, as the concentration of the target antigen 20 increases, the amount of the switching binder 130 separated from the second conjugate (b2) increases, thereby increasing the fluorescence amount of the solution.

In an embodiment, in the assay step (S300 "), a test kit capable of identifying an antibody and an antigen through the amount of the switching binder 130 separated according to an increase in the concentration of the target antigen 20 may be provided. According to an embodiment of the present disclosure, the test kit using the switching binder 100 may be performed according to an immunoassay method. For example, the test kit may include a luminex assay, a protein microarray assay, an ELISA assay, a capture-ELISA assay, an ELISPOT assay, a radioimmunoassay (RIA), a lateral flow immunoassay (LFIA), a flow-through immunoassay (FTIA), a protein microfluidic immunoassay, a protein capillary electrophoresis (CE), an immuno-PCR assay, an electrochemical biosensor, a radioimmunoprecipitation assay, an immunoprecipitation assay, an immunohistochemical staining assay, an Ouchterlony immunodiffusion assay, an inhibition or competition assay, a sandwich assay, a flow cytometry, an immunoelectrophoretic assay, a tissue immunostaining assay, a complement fixation assay, a FACS assay, a protein chip assay, an immunofluorescence staining assay, and an immunoaffinity purification assay methods. The above-described assay methods are merely exemplary and are not limited thereto. With respect to the immunoassay kits, descriptions of commercially available assay methods may be applied. In another embodiment, the test kit may include the switching binder 100 labeled with a natural or artificial labeling material 200. The description of the labeling material 200 may refer to FIG. 3.

In an embodiment, in the assay step (S300"), a test kit that can measure the amount of the switching binder 130 separated according to the increase in the concentration of the target antigen 20 and measure the binding strength of the target antigen 20 to the antibody 10 can be provided. In order to measure the binding strength, the antigen-binding coefficient (K_{d}) of the antibody 10 may be measured. The antigen-binding coefficient (K_{d}) may be an equilibrium dissociation constant indicating the strength of the antigen-antibody binding interaction. The higher the antigen-binding coefficient (K_{d}), the higher the binding affinity between the target antigen 20 and the antibody 10. As the concentration of the antigen increases, the separated switching binder 130 undergoes a fluorescence labeling reaction to thereby increase the fluorescence amount of the solution, and accordingly, it can be calculated through measurement for each concentration. Accordingly, the strength of binding can be known from the difference between the antigen-antibody binding coefficient and the binding coefficient measured by a conventional method through the amount of the switching binder 100 released by the antigen-antibody binding.

In an embodiment, the test kit may provide a test kit capable of measuring the binding strength of the antigen 20 to the antibody 10 in a solution in which the antibody 10 is not immobilized. According to an embodiment of the present disclosure, a method of measuring the antigen-binding coefficient (K_{d}) when the antibody 10 contained in the solution binds the target antigen 20 without immobilizing the antibody 10 on the solid support 30 is used, which omits the step of immobilizing the antibody 10. Thus, the reliability and accuracy of the measurement are improved. Therefore, it is also possible to develop a test kit capable of the one-step immunoassay using the switching binder 100 that reversibly binds to the framework regions (FR, FR') of the antibody, the antibody being in solution through an assay method of measuring the antigen-binding coefficient (K_{d}), which is the binding strength with the antigen.

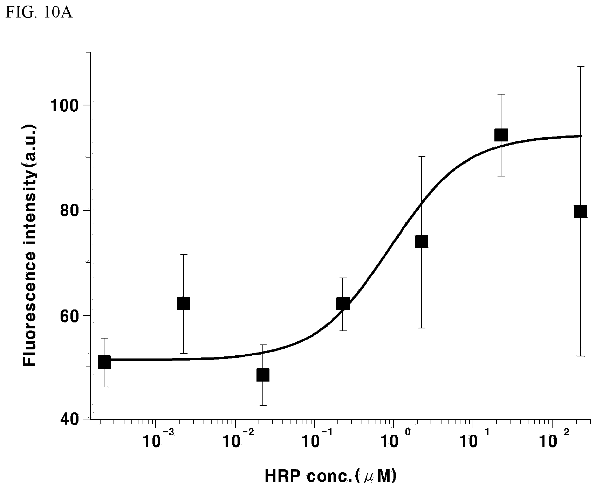

In another embodiment, the test kit may include the switching binder 100 labeled with a natural or artificial labeling material 200. The description of the switching binder 100 and the labeling material 200 may refer to FIGS. 1A to 3. Assay results for the assay method disclosed in FIG. 4C will be described in detail with reference to FIGS. 10A to 10D.

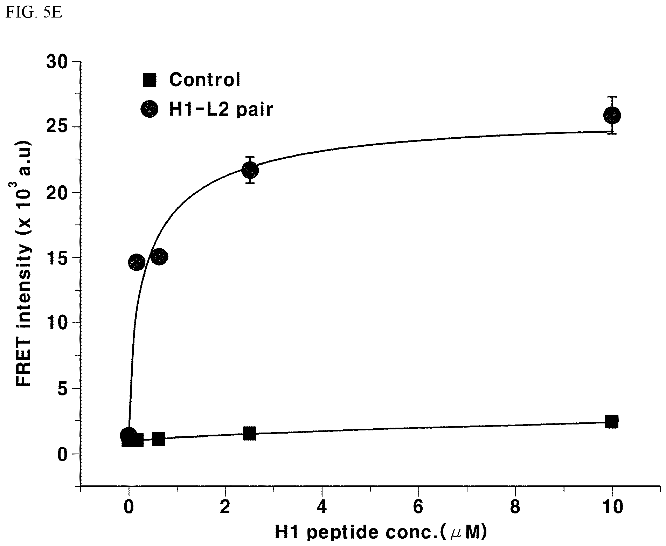

FIG. 5A shows the binding between the switching binder of SEQ ID NO. 2 (H2) and the switching binder of SEQ ID NO. 3 (L1); FIG. 5B shows the specific binding between the amino acid sequence of the switching binder of SEQ ID NO. 2 (H2) and the switching binder of SEQ ID NO. 3 (L1); FIG. 5C is a graph showing the interaction between the switching binder of SEQ ID NO. 2 (H2) and the switching binder of SEQ ID NO. 3 (L1) for each site; FIG. 5D is a diagram showing the hydrophobicity index between the switching binder of SEQ ID NO. 2 (H2) and the switching binder of SEQ ID NO. 3 (L1); and FIG. 5E is a graph of FRET assay between the switching binder of SEQ ID NO. 2 (H2) and the switching binder of SEQ ID NO. 3 (L1).

Referring to FIG. 5A, in an embodiment, a three-dimensional structure is formed between the switching binder of SEQ ID NO. 2 (H2) and the switching binder of SEQ ID NO. 3 (L1) to form a mutual bond. That is, the switching binder of SEQ ID NO. 2 (H2) and the switching binder of SEQ ID NO. 3 (L1) do not interact in a straight line. Accordingly, the lower end of the switching binder of SEQ ID NO. 2 (H2) and the upper end of the switching binder of SEQ ID NO. 3 (L1) are interacting, and the upper end of the switching binder of SEQ ID NO. 2 (H2) and the lower end of the switching binder of SEQ ID NO. 3 (L1) are interacting. In another embodiment, the binding of the adjacent region between the switching binder of SEQ ID NO. 2 (H2) and the switching binder of SEQ ID NO. 3 (L1) may act at a short distance of about 4 Å to 8 Å, preferably about 3 Å to 6 Å. Similarly in the framework region (FR) that self-associates and interacts with the switching binder of SEQ ID NO. 2 (H2) or the switching binder of SEQ ID NO. 3 (L1) through the linking distance between the switching binders (H2, L1), structural characteristics of adjacent sites may act at a short distance of about 4 Å to about 8 Å, preferably about 3 Å to about 6 Å.

Referring to FIG. 5B, in an embodiment, a portion of the amino acid sequence of the switching binder of SEQ ID NO. 2 (H2) and the switching binder of SEQ ID NO. 3 (L1) may cause a hydrogen bond. Specifically, as shown in FIG. 5B, the switching binder of SEQ ID NO. 2 (H2) includes the sequence of V-Y-Y-C-A-R-E-P-T-G-T-G-I-Y-F-D-V-W-G-K, and the switching binder of SEQ ID NO. 3 (L1) may include the sequence of T-Y-L-E-W-Y-P-Q-K-P-G-Q-S-P-K-L-L-I-Y-K.

In an embodiment, the amino acid sequence may include a first antibody-binding portion (AB1) or a second antibody-binding portion (AB2) that mainly binds. For example, as the first antibody-binding portion (AB1), the third amino acid sequence Y and the 18th amino acid sequence W among the amino acid sequence of the peptide sequence of SEQ ID NO. 2 (H2) interact through the amino acid side chain functional group. The 13th amino acid sequence I can cause interaction with the polar region of the peptide bond.

In another embodiment, the amino acid sequence extracted from the light chain may include a second antibody-binding portion (AB2) that binds to the framework region of the heavy chain (VH) constituting the antigen-binding portion together with the light chain. For example, as the second antibody-binding portion (AB2), the 6th amino acid sequence Y and the 8th amino acid sequence Q of the amino acid sequence of the switching binder sequence of SEQ ID NO. 3 (L1) interact through a side chain functional group. The 14th amino acid sequence P and the 16th amino acid L interact with the polar region of the peptide bond. Accordingly, as shown in the figure, amino acids underlined in red may cause an interaction through a side chain functional group, and amino acids indicated in blue may cause interaction with a polar site of a peptide bond. However, this is non-limiting, and as described above in FIG. 1A, and may include not only the structural characteristics of the antigen-binding portion of the antibody that cross-links with each other but also the case where the amino acid sequence is extracted to mutually bind by various bonds such as polar or nonpolar bonds.

Referring to FIGS. 5C and 5D, in an embodiment, the first antibody-binding portion (AB1) and the second antibody-binding portion (AB2) between the switching binder of SEQ ID NO. 2 (H2) and the switching binder of SEQ ID NO. 3 (L1) may have hydrophobicity indexes according to the amino acid side chain. In FIG. 5C, if the hydrophobicity index is positive, it is located inside the protein, while if the hydrophobicity index is negative, it can be located outside the protein because it is water-soluble. In addition, the hydrophobic portion may cause interaction with the hydrophobic portion and the hydrophilic portion with the hydrophilic portion. The upper end of the switching binder of SEQ ID NO. 2 (H2) and the switching binder of SEQ ID NO. 3 (L1) are hydrophilic, and the lower end is hydrophobic.

Accordingly, the hydrophilic moiety and the hydrophobic moiety interact with each other, and the lower end of the switching binder of SEQ ID NO. 2 (H2) and the upper end of the switching binder of SEQ ID NO. 3 (L1), and the upper end of the switching binder of SEQ ID NO. 2 (H2) and the lower end of the switching binder of SEQ ID NO. 3 (L1) can interact in a three-dimensional structure. Thus, the switching binder of SEQ ID NO. 2 (H2) can interact with the LAR region (LAR) of the light chain (V_{L}) comprising the same amino acid sequence as the switching binder of SEQ ID NO. 3 (L1). The switching binder of SEQ ID NO. 3 (L1) can interact with the HAR region (HAR) of the heavy chain (V_{H}) comprising the same amino acid sequence as the switching binder of SEQ ID NO. 2 (H2). If the amino acids at the four hydrogen bond positions between the switching binder of SEQ ID NO. 2 (H2) and the switching binder of SEQ ID NO. 3 (L1) observed through the three-dimensional structure are modulated in consideration of the three-dimensional structure, the binding strength of the antibody-binding portion 15 can be adjusted.

Referring to FIG 5E, an embodiment provides FRET test results showing the mutual binding between the antibody binding switching binders (100). This indicates the mutual binding between the switching binder of SEQ ID NO. 2 (H2) and the switching binder of SEQ ID NO. 3 (L1). Accordingly, it can be confirmed that the switching binder of SEQ ID NO. 2 (H1) can self-bind and interact with the LAR region of the light chain (V_{L}), and the switching binder of SEQ ID NO. 3 (L1) can self-bind and interact with the HAR region of the heavy chain (V_{H}). Through this, the switching binder 100 having an amino acid sequence extracted from the heavy chain (VH) can bind to the antigen-binding portion 15 including the framework region (FR) of the light chain (V_{L}), and the switching binder 100 having an amino acid sequence extracted from the light chain (V_{L}) can bind to the antigen-binding portion 15 including the framework region (FR) of the heavy chain (V_{H}). Through this mutual binding property, the switching binder 100 binds to the antigen-binding portion 15 including the framework regions (FR, FR'), and thus the same effect as the interaction between the framework regions (FR, FR') can be obtained.

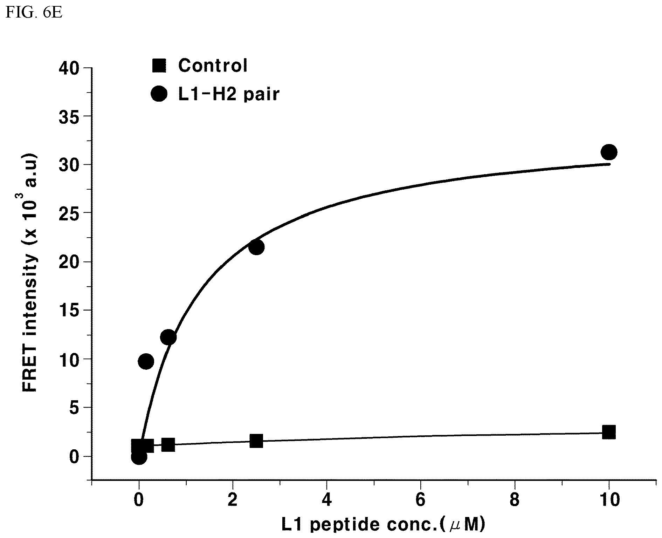

FIG. 6A shows the binding between the switching binder of SEQ ID NO. 1 (H1) and the switching binder of SEQ ID NO. 4 (L2); FIG. 6B shows the specific binding between the amino acid sequence of the switching binder of SEQ ID NO. 1 (H1) and the switching binder of SEQ ID NO. 4 (L2); FIG. 6C is a graph showing the interaction between the switching binder of SEQ ID NO. 1 (H1) and the switching binder of SEQ ID NO. 4 (L2) for each site; FIG. 6D is a diagram showing the hydrophobicity index between the switching binder of SEQ ID NO. 1 (H1) and the switching binder of SEQ ID NO. 4 (L2); and FIG. 6E is a graph of FRET assay between the switching binder of SEQ ID NO. 1 (H1) and the switching binder of SEQ ID NO. 4 (L2).

Referring to FIG. 6A, in an embodiment, a three-dimensional structure is formed between the switching binder of SEQ ID NO. 1 (H1) and the switching binder of SEQ ID NO. 4 (L2) to interact with each other. That is, the switching binder of SEQ ID NO. 1 (H1) and the switching binder of SEQ ID NO. 4 (L2) do not interact in a straight line. Accordingly, the lower end of the switching binder of SEQ ID NO. 1 (H1) and the upper end of the switching binder of SEQ ID NO. 4 (L2) are interacting, and the upper end of the switching binder of SEQ ID NO. 1 (H1) and the lower end of the switching binder of SEQ ID NO. 4 (L2) are interacting. In another embodiment, the binding of the adjacent region between the switching binder of SEQ ID NO. 1 (H1) and the switching binder of SEQ ID NO. 4 (L2) may act at a short distance of about 4 Å to 8 Å, preferably about 3 Å to 6 Å. Similarly in the framework regions (FR, FR') that self-associates and interacts with the switching binder of SEQ ID NO. 1 (H1) or the switching binder of SEQ ID NO. 4 (L2) through the linking distance between the switching binders (HI, L2), structural characteristics of adjacent sites may act at a short distance of about 4 Å to about 8 Å, preferably about 3 Å to about 6 Å.

Referring to FIG. 6B, in an embodiment, a portion of the amino acid sequence of the switching binder of SEQ ID NO. 1 (H1) and the switching binder of SEQ ID NO. 4 (L2) may cause a hydrogen bond. Specifically, the switching binder of SEQ ID NO. 1 (H1) may include the sequence of S-Y-W-I-H-W-V-K-Q-R-P-G-Q-G-L-E-W-I-G-E, and the switching binder of SEQ ID NO. 4 (L2) may include the sequence of V-Y-Y-C-F-Q-G-S-H-V-P-F-T-K.

In an embodiment, similar to FIG. 5B, the amino acid sequence may include a first antibody-binding portion (AB1') or a second antibody-binding portion (AB2') that mainly binds. For example, as the first antibody-binding portion (AB1'), the 9th amino acid sequence Q among the amino acid sequence of the switching binder of SEQ ID NO. 1 (H1) interacts through the amino acid side chain functional group. The 17th amino acid sequence W can cause interaction with the polar region of the peptide bond. In another embodiment, a second antibody-binding portion (AB2') may be included. For example, as the second antibody-binding portion (AB2'), the 3rd amino acid sequence Y among the amino acid sequence of the switching binder of SEQ ID NO. 4 (L2) interacts through the amino acid side chain functional group. The 12th amino acid sequence F can cause interaction with the polar region of the peptide bond. Accordingly, as shown in the figure, amino acids underlined in red may cause an interaction through a side chain functional group, and amino acids indicated in blue may cause interaction with a polar site of a peptide bond.

Referring to FIGS. 6C and 6D, in an embodiment, the first antibody-binding portion (AB1') and the second antibody-binding portion (AB2') between the switching binder of SEQ ID NO. 1 (H1) and the switching binder of SEQ ID NO. 4 (L2) may have hydrophobicity indexes according to the amino acid side chain. In FIG. 6C, if the hydrophobicity index is positive, it is located inside the protein, while if the hydrophobicity index is negative, it can be located outside the protein because it is water-soluble. In addition, the hydrophobic portion may cause interaction with the hydrophobic portion and the hydrophilic portion with the hydrophilic portion. The upper end of the switching binder of SEQ ID NO. 1 (H1) and the switching binder of SEQ ID NO. 4 (L2) are hydrophilic, and the lower end is hydrophobic.

Accordingly, the hydrophilic moiety and the hydrophobic moiety interact with each other, and the lower end of the switching binder of SEQ ID NO. 1 (H1) and the upper end of the switching binder of SEQ ID NO. 4 (L2), and the upper end of the switching binder of SEQ ID NO. 1 (HI), and the lower end of the switching binder of SEQ ID NO. 4 (L2) can interact in a three-dimensional structure. Accordingly, the switching binder of SEQ ID NO. 1 (H1) can interact with the LBR region of the light chain (V_{L}), and the switching binder of SEQ ID NO. 4 (L2) can interact with the HBR region of the heavy chain (V_{H}). If the amino acid residues of the first antibody-binding portion (AB1') or the second antibody-binding portion (AB2') are modulated in consideration of the three-dimensional structure of amino acids at two hydrogen bond positions between the switching binder of SEQ ID NO. 1 (H1) and the switching binder of SEQ ID NO. 4 (L2) observed through the three-dimensional structure, the binding strength of the antibody-binding portion can be controlled. However, the present disclosure is not limited thereto, and any combination between the switching binder 100 having an amino acid sequence extracted from a site where the heavy chain (V_{H}) and the light chain (V_{L}) bind by hydrogen bond and interact with each other and the antigen-binding portion 15 comprising the framework region (FR) of the heavy chain (V_{H}) or light chain (V_{L}) can be included.

Referring to FIG 6E, an embodiment provides FRET test results showing the mutual binding between the antibody binding switching binders. This indicates the mutual binding between the switching binder of SEQ ID NO. 1 (H1) and the switching binder of SEQ ID NO. 4 (H2). Through this, it can be confirmed that the switching binder of SEQ ID NO. 1 (H1) can interact with the LBR region of the light chain (V_{L}), and the switching binder of SEQ ID NO. 4 (L2) can interact with the HBR region of the heavy chain (V_{H}). Through this property, the switching binder 100 binds to the framework region (FR), and thus the same effect as the interaction between the framework regions (FR) can be obtained.

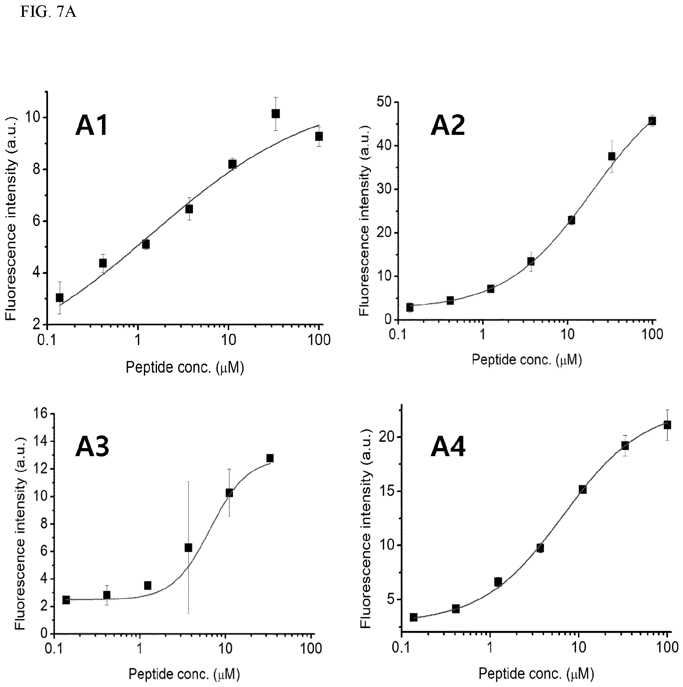

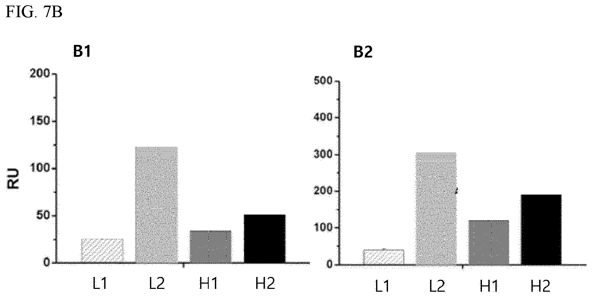

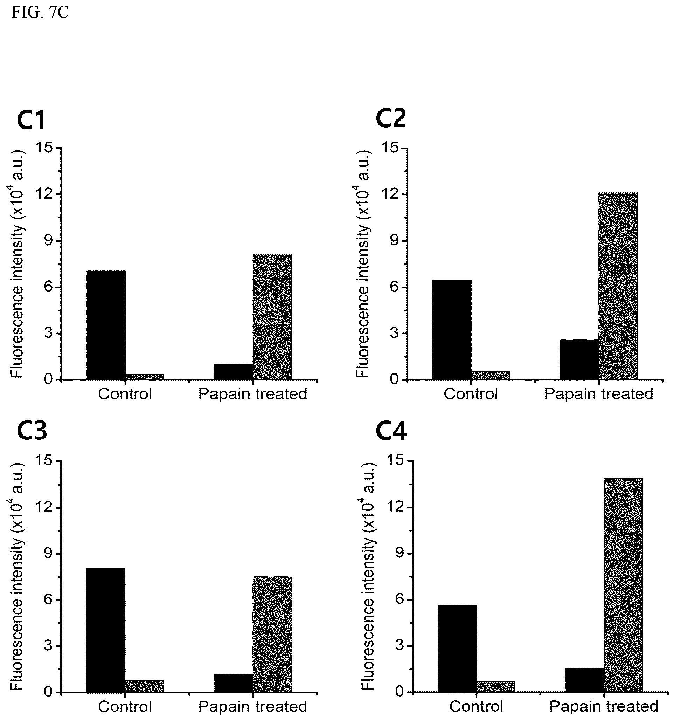

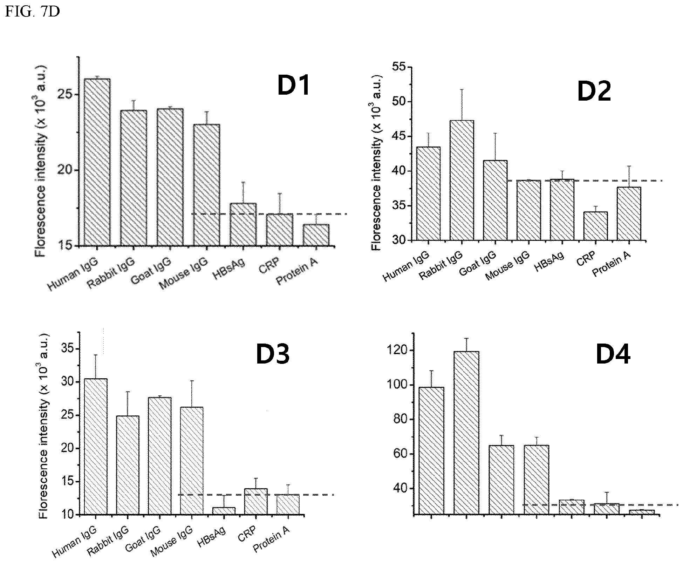

FIG. 7A shows the antibody-binding strength measurement results for each switching binder 100; FIG. 7B shows SPR analysis results for the binding strength of the antibody binding switching binder 100 according to the concentration; FIG. 7C shows the results for the antibody 10 Fab site binding of the switching binder 100 bound to the antibody; and FIG. 7D shows a comparative analysis graph of the antibody 10 Fab site binding of the switching binder 100 bound to the antibody 10 with other antigens and proteins.

Referring to FIG. 7A, based on the concentration of the switching binder 100, it shows a graph (A1) showing the amount of fluorescence of the switching binder of SEQ ID NO. 1 (HI); a graph (A2) showing the fluorescence amount of the switching binder of SEQ ID NO. 2 (H2), a graph (A3) showing the fluorescence amount of the switching binder of SEQ ID NO. 3 (L1), and a graph (A4) showing the fluorescence amount of the switching binder of SEQ ID NO. 4 (L2).

In an embodiment, it can be confirmed that as the concentration of the switching binder 100 of the present disclosure increases, the intensity of the fluorescence intensity increases. Through this, the switching binder 100 of the present disclosure binds to the antibody 10 and can be used for quantification of the bound switching binder 100. In another embodiment, the antigen-binding coefficient (K_{d}) may be measured by measuring the fluorescence amount for each concentration of the switching binder 100. The antigen-binding coefficient of the switching binders 100 of each SEQ ID NO. 1 to SEQ ID NO. 4 was calculated by K_{d}(H1) =1.42×10⁻⁶ M, K_{d}(H2) =1.94×10⁻⁵ M, K_{d}(L1) =6.62×10⁻⁶ M and K_{d}(L2) =6.68 × 10⁻⁶ M.

Through this, there is an advantage in calculating and quantifying the relative binding strength of the target antigen 20 to the antibody 10 for each switching binder. A specific experimental method of fluorescence analysis will be described later in Example 3. However, the antigen-binding coefficient (K_{d}) shown in FIG. 7A represents a value for the antibody 10 immobilized by the support 30 but is not limited thereto. It is also possible to measure the antigen-binding coefficient (K_{d}) when the antibody 10 binds the target antigen 20 in the solution, which will be described in detail with reference to FIGS. 10A to 10D.

Referring to FIG. 7B, in an embodiment, a result of a surface plasmon resonance (SPR) analysis may be confirmed. Surface Plasmon Resonance (SPR) uses a phenomenon that when a material binds to the surface of a metal thin film such as gold, the refractive index of the metal surface changes. When the light is irradiated, the concentration of the material bound to the metal surface can be measured by examining the resonance angle at which the SPR phenomenon occurs among the reflected light. The switching binder 100 for each concentration, in this experimental example, shows the result value of the SPR signal treated with the peptide among the binders.

Specifically, in order to confirm the SPR signal result, the gold surface was treated overnight with 1 mM mercaptoundecanoic acid (MUA) and surface-modified prior to immobilization of the antibody 10. Carbodiimide/N-Hydroxysuccinimide (EDC/NHS, 0.4 M/0.1 M) was mixed in a 1:1 ratio and treated for 10 minutes to activate functional groups. Antibody immobilization was prepared by treating an anti-HRP antibody with an antibody concentration of 50 µg/mL for 1.5 hours. Thereafter, it was used after blocking with an ethanolamine concentration of 1 M for 10 minutes. All of the switching binders 100 were treated by incubation for 30 minutes using two concentrations of 10 and 20 µM, respectively. The SPR signal can be measured as an average value by repeating the routine of measuring the SPR signal for 3 minutes while the solution is stopped after flowing 1% Tween-20 washing solution for 1 minute three times.

However, the embodiment is not limited to the embodiment of the present disclosure and may include any method capable of measuring the binding strength of the switching binder 100. In the graph, the SPR signal of the peptide of SEQ ID NO. 1 (HI), the SPR signal of the peptide of SEQ ID NO. 2 (H2), the SPR signal of the peptide of SEQ ID NO. 3 (L1), and the SPR signal of the peptide of SEQ ID NO. 4 (L2), the low concentration of 10 µM is shown in the graph on the left (B1), and the high concentration of 20 µM is shown in the graph on the right (B2). As a result of peptide treatment at a low concentration of 10 µM, shown in the graph on the left (B1), the SPR signal was calculated as the peptide of SEQ ID NO. 1 (H1) = 34.4±3.3 (9.6%) RU, the peptide of SEQ ID NO. 2 (H2) = 51.5±5.6 (10.9%) RU, the peptide of SEQ ID NO. 3 (L1) = 25.3±3.2 (12.5%) RU, and the peptide of SEQ ID NO. 4 (L2) = 124.6±16.9 (13.6%) RU.

As a result of peptide treatment at a high concentration of 20 µM shown in the graph (B2) on the right, the SPR signal can confirm the analysis results of the peptide of SEQ ID NO. 1 (H1) = 121.4±3.7 (3.1%) RU, the peptide of SEQ ID NO. 2 (H2) = 192.0±1.8 (0.9%) RU, the peptide of SEQ ID NO. 3 (L1) = 40.4±3.2 (7.9%) RU, and the peptide of SEQ ID NO. 4 (L2) = 418.2±26.5 (6.4%) RU. Through this, it can be seen that the peptides of SEQ ID NO. 1 to SEQ ID NO. 4 synthesized by analyzing the amino acid sequence of the antigen-binding portion of the antibody bind to the antibody, and among them, it can be seen that the peptide of SEQ ID NO. 4 binds the most.

Referring to FIGS. 7C and 7D, they show a graph (C1, D1) showing the amount of fluorescence of the switching binder of SEQ ID NO. 1 (HI); a graph (C2, D2) showing the fluorescence amount of the switching binder of SEQ ID NO. 2 (H2), a graph (C3, D3) showing the fluorescence amount of the switching binder of SEQ ID NO. 3 (L1), and a graph (C4, D4) showing the fluorescence amount of the switching binder of SEQ ID NO. 4 (L2). In an embodiment, it can be seen that the switching binder 100 of SEQ ID NOs. 1 to 4 binds only to the Fab site including the antigen-binding portion 15 among the fragments of the antibody 10.

Referring back to FIG. 7C, in an embodiment, the switching binder 100 may bind to the antigen-binding portion 15 of the antibody 10. Specifically, a fluorescence-labeled switching binder 100 was bound to the antibody 10 immobilized on the solid support 30. Antibody immobilization was prepared by treating the anti-HRP antibody 10 with an antibody concentration of 10 µg/mL for two hours and was used after blocking with a BSA concentration of 8 mg/mL for 1 hour. The switching binder 100 of SEQ ID NOs. 1 to 4 was incubated for 1 hour at a concentration of 10 µM of the switching binder 100. After treatment with papain at a concentration of 250 µg/mL for 2 hours at 37°C for decomposition of the Fab region and Fc region of the antibody 10, the change in fluorescence amount in the solution is measured, and the amount of fluorescence remaining on the solid support 30 was measured separately.

In an embodiment, the amount of fluorescence in the bottom (A) and the amount of fluorescence in the solution (B) can be confirmed. After treatment with papain, a proteolytic enzyme that decomposes the Fab site and the Fc region of the antibody 10, fluorescence was measured in the switching binder 100. When the amount of fluorescence is papain treated, it can be confirmed that the amount of fluorescence at the Fab site freed in solution (B) is higher than that at the bottom where the Fc region is fixed (A). That is, before treatment with papain, an antibody degrading enzyme, for the switching binder 100 of SEQ ID NOs. 1 to 4, compared to a large amount of fluorescence observed only in the solid support 30 on which the antibody is fixed, in the case of papain treatment, the fluorescence was greatly reduced in the solid support on which the antibody 10 was immobilized, and it was largely observed in the solution. Through this, it can be seen that the switching binder 100 of SEQ ID NOs. 1 to 4 is bound to the Fab region that is decomposed into papain and distributed in the solution and does not bind to the Fc region fixed to the solid support 30.

Referring back to FIG. 7D, in an embodiment, the switching binder 100 of SEQ ID NOs. 1 to 4 may selectively bind only to the antibody 10. Specifically, human IgG, rabbit IgG, goat IgG, and mouse IgG were treated overnight at an antibody concentration of 100 µg/mL for immobilization of the antibody 10 and protein on the solid support 30. The immobilized protein was prepared by overnight treatment with HBsAg, CRP, and protein A at a concentration of 100 µg/mL, respectively. Then, it was used after blocking for 1 hour at a BSA concentration of 10 mg/mL. The switching binders 100 of SEQ ID NOs. 1 to 4 were treated by incubation for 1 hour at a concentration of 20 µM, respectively.

Referring to FIG. 7E, it was confirmed that the fluorescence-labeled peptides of SEQ ID NOs. 1 to 4 showed high fluorenscence levels in the antibodies such as human IgG, rabbit IgG, goat IgG, and mouse IgG compared to the antigens HBsAg and CRP and protein A. This confirms that the switching binder 100 of SEQ ID NO. 1 to SEQ ID NO. 4 selectively binds to the antibody 10.

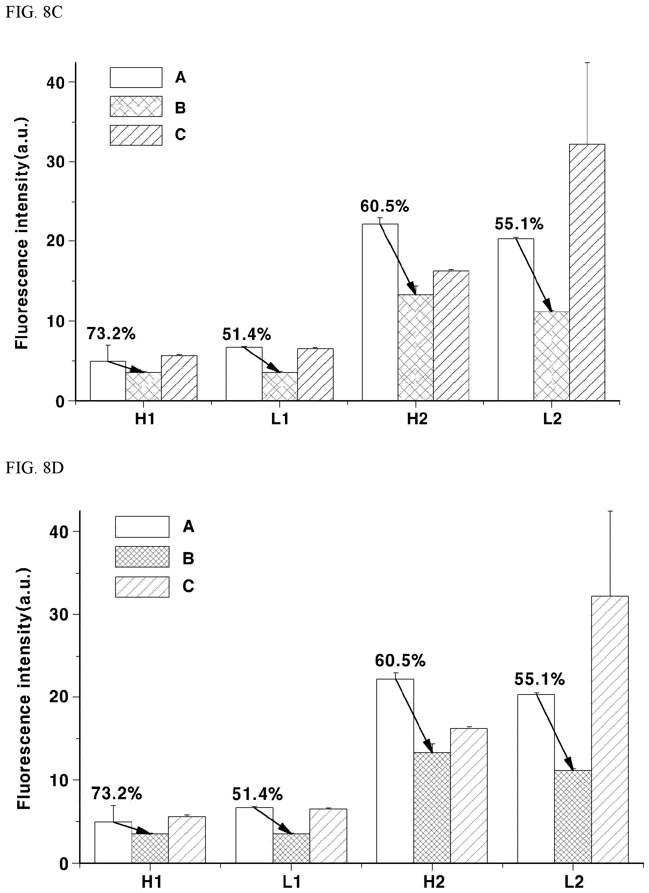

FIGS. 8A to 8D are graphs showing the assay results of the target antigen 20 according to the assay method of FIG. 4A in an embodiment of the present disclosure, in which FIG. 8A shows when the antibody 10 is an anti-HRP antibody 10, and the target antigen 20 is HPR; FIG. 8B shows when the antibody 10 is an anti-hCG antibody 10, and the target antigen 20 is hCG; FIG. 8C shows when the antibody 10 is an anti-CRP antibody 10, and the target antigen 20 is CRP; and FIG. 8D shows when the antibody 10 is an anti-HBsAg antibody 10, and the target antigen 20 is HBsAg.

With reference to FIG. 8A, anti-HRP antibody was treated and fixed overnight on a solid support at a concentration of 1 µg/ml, and the switching binder 100 of SEQ ID NOs. 1 to 4 were each treated at a concentration of 10 µM (100 µl/well) for 2 hours. At this time, after measuring the fluorescence of the bottom, the antigen HRP as an analyte for an IgG antibody of rabbit was treated at a concentration of 10 µg/ml for 1 hour. With reference to FIG. 8B, anti-HCG antibody was treated and fixed overnight on a solid support at a concentration of 1 µg/ml, and the switching binder 100 of SEQ ID NOs. 1 to 4 were each treated at a concentration of 20 µM (100 µl/well) for 2 hours. At this time, after measuring the fluorescence of the bottom, the antigen HCG as an analyte for an IgG antibody of a mouse was treated at a concentration of 100 µg/ml for 2 hours.

With reference to FIG. 8C, anti-CRP antibody was treated and fixed overnight on a solid support at a concentration of 1 µg/ml, and the switching binder 100 of SEQ ID NOs. 1 to 4 were each treated at a concentration of 20 µM (100 µl/well) for 2 hours. At this time, after measuring the fluorescence of the bottom (A), the antigen HRP as an analyte for an IgG antibody of rabbit was treated at a concentration of 10 µg/ml for 1 hour. With reference to FIG. 8D, anti-HBsAg antibody was treated and fixed overnight on a solid support at a concentration of 1 µg/ml, and the switching binder 100 of SEQ ID NOs. 1 to 4 were each treated at a concentration of 20 µM (100 µl/well) for 2 hours. At this time, after measuring the fluorescence of the bottom (A), the antigen HBsAg as an analyte for an IgG antibody of a goat was treated at a concentration of 10 µg/ml for 1 hour.

Referring to FIGS. 8A to 8D, the switching binder 100 was tested using the switching binder of SEQ ID NOs. 1 to 4, in particular, peptides in the binder 100. In FIGS. 8A to 8D, graph A may be a measurement value of the switching binder 110 bound to the antibody 10 in the pretreatment step (S100); graph B may be a measurement value of the residual amount of the switching binder 120 bound to the framework region (FR) in the assay step S300; graph C may be a measurement of the switching binder 130 separated from the framework region (FR); for example, the intensity of the labeling reaction exhibited by the labeling material 200 bound to the switching binder 100 was measured and quantified. The labeling reaction may be a fluorescence reaction. The materials described above are non-limiting examples and the present disclosure is not limited thereto.

Referring to FIGS. 8A to 8D, it can be seen that the remaining amount (B) of the switching binder 120 bound to the framework region (FR) was reduced compared to the amount (A) of the switching binder 110 bound to the antibody 10 in all of the switching binder of SEQ ID NO. 1 (HI), the switching binder of SEQ ID NO. 2 (H2), the switching binder of SEQ ID NO. 3 (L1), and the switching binder of SEQ ID NO. 4 (L2). In addition, it can be seen that the amount (C) of the switching binder 130 separated from the framework region (FR) is measured. As a result, it can be seen that the reduction amount of the remaining amount (B) of the switching binder 120 bound to the framework region (FR) relative to the amount (A) of the switching binder 110 bound to the antibody 10 is greater than the amount (C) of the switching binder 130 separated from the framework region (FR).

This is because the amount (A) of the switching binder 110 bound to the antibody 10 is the intensity of fluorescence observed on the surface of the support 30, but the amount (C) of the switching binder 130 separated from the framework region (FR) is the intensity of fluorescence observed in the solution phase. Accordingly, it can be seen that when the target antigen 20 is bound to the antibody 10, the switching binder 110 bound to the antibody 10 is separated from the framework region (FR) and released into a solution phase. According to an embodiment of the present disclosure, as described above, it is possible to quantify the target antigen 20 by the difference between the amount of the switching binder 110 bound to the antibody 10 measured in the pretreatment step (S100) and the amount of the switching binder 120 in the remaining amount bound to the framework region (FR) in the assay step (S300). At the same time, it is possible to quantify the target antigen 20 using the amount of the switching binder 130 separated from the framework (FR), and thus the assay of the target antigen 20 can be performed in two tracks, and the speed, accuracy, and reliability of the assay can be improved.

In addition, in order to calculate inter-measurement reproducibility for quantitative analysis, the experiment was repeated eight times at an HBsAg concentration of 3.4 µg/ml using the binder of SEQ ID NO. 3 (L1), and then the HBsAg concentration was normalized to the baseline value of zero (0). At this time, the measured value at the corresponding concentration was calculated as 3.1 ± 9.3% (n=8). The above results indicate that a sample containing the analyte antigen 20 HBsAg is treated with a goat IgG antibody, and the measurement of the analyte by one-step immunoassay without washing shows high reproducibility.

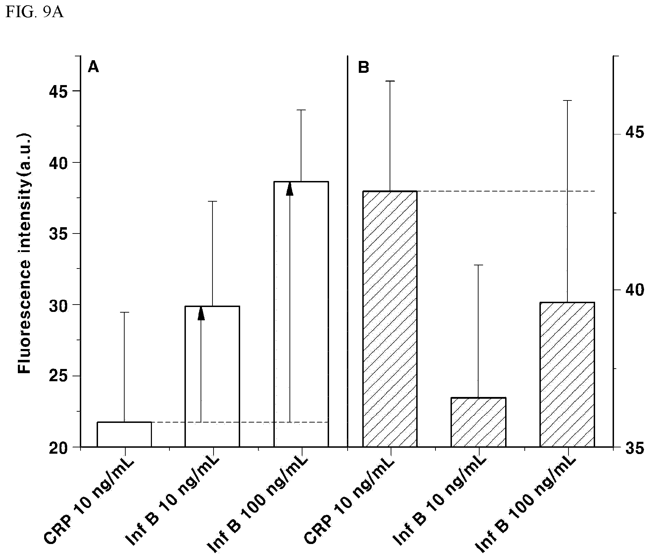

FIGS. 9A and 9B are assay graphs showing the assay results of the target antigen 20 according to the assay method of FIG. 4B.

Referring to FIGS. 9A and 9B, in an embodiment, FIG. 9A shows a bead-based antibody binding one-step immunoassay result using a switching binder of SEQ ID NO. 1 (HI), and FIG. 9B shows a bead-based antibody binding one-step immunoassay result using a switching binder of SEQ ID NO. 3 (L1).

In an embodiment, the anti-influenza B antibody 10 is fixed to the beads in the pretreatment step (S100'), and in the antigen processing sub-step (S200'), the analyte may contain CRP at a concentration of 10 ng/mL or influenza B at a concentration of 10 ng/mL. The graph on the left (A) is a graph quantifying the switching binder 130 isolated from the framework regions (FR, FR') of the analytes including different target antigens 200, that is, CRP and influenza B, and the graph on the right (B) is a graph quantifying the residual amount of the switching binder 120 bound to the framework regions (FR, FR'). The materials described above are non-limiting examples and the present disclosure is not limited thereto.

In an embodiment, it can be seen that when the analyte contains CRP compared to when the analyte contains influenza B, the amount of the separated switching binder 130 is small and the remaining amount of the switching binder 120 is large. This is because when the analyte is influenza B, the influenza B reacts with the anti-influenza B antibody 10 and the switching binder 100 dissociates from the framework regions (FR, FR') of the antibody 10. On the other hand, when the analyte contains CRP, the CRP does not specifically bind to the anti-influenza B, such that the switching binder 100 does not dissociate from the framework regions (FR, FR').

In an embodiment, the higher the concentration of the target antigen 20 in the analyte, the greater the amount of the separated switching binder 130. In addition, the higher the concentration of the target antigen 20 in the analyte, the smaller the remaining amount of the switching binder 120. Referring to FIGS. 9A and 9B, when the analytes equally contain influenza B as the target antigen 20, the higher the concentration of influenza B, the greater the amount of the isolated switching binder 130. For example, when the concentration of influenza B included in the analyte is 100 ng/mL, the amount of the isolated switching binder 130 may be greater than the concentration of 10 ng/mL. This is because the higher the concentration of the target antigen 20, the greater the amount of the target antigen 20 specifically reacts with the antibody 10, and the greater the amount of the switching binder 100 is dissociated from the framework regions (FR, FR').

According to an embodiment of the present disclosure, by measuring the amount of the separated switching binder 130 and/or the remaining amount of the switching binder 120, the target antigen 20 included in the analyte can be identified, but also an assay method capable of quantitative or even concentration measurement may be provided. Through this, in FIG. 9A, it can be confirmed that a bead-based one-step immunoassay using the switching binder of SEQ ID NO. 1 (H1) is possible and in FIG. 9B, it can be confirmed that a bead-based one-step immunoassay using the switching binder of SEQ ID NO. 3 (L1) is possible. Therefore, quantitative immunoassays are possible using beads as well as solid supports. However, this is non-limiting and is not limited to the amino acid sequences of SEQ ID NO. 1 and SEQ ID NO. 3.

FIGS. 10A to 10D are assay graphs showing the analysis results of the target antigen 20 according to the assay method of FIG. 4C in an embodiment of the present disclosure.

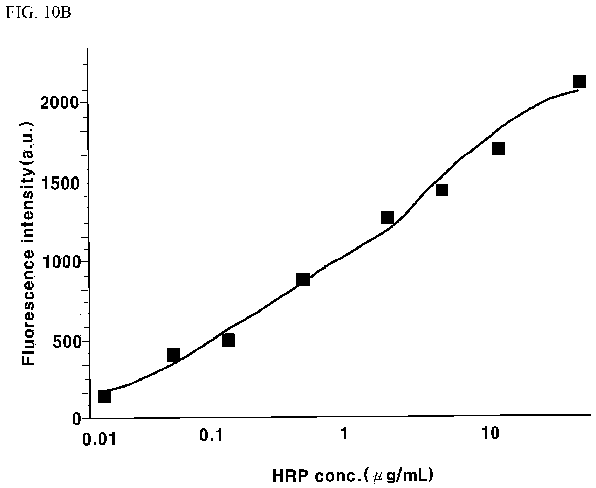

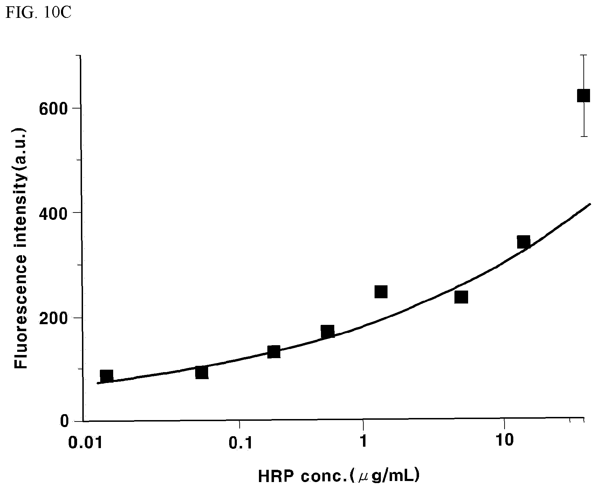

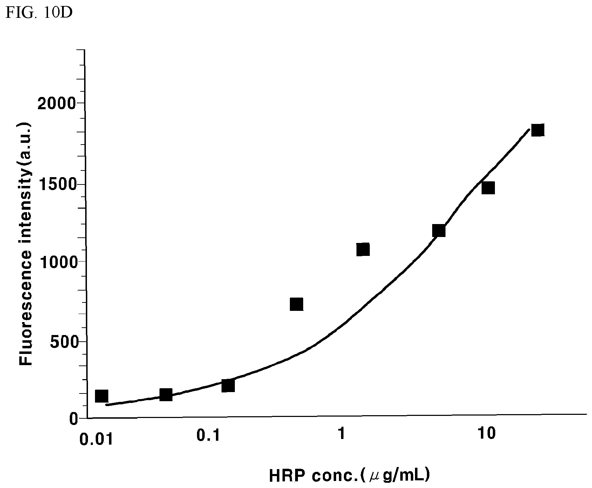

Referring to FIGS. 10A to 10D, the switching binder 100 uses a switching binder having the amino acid sequence of SEQ ID NOs. 1 to 4, in which the results of measuring the antigen-binding coefficient of the antibody in solution are shown using a switching binder of SEQ ID NO. 3 (L1) (FIG. 10A), the switching binder of SEQ ID NO. 4 (L2) (FIG. 10B), the switching binder of SEQ ID NO. 1 (H1) (FIG. 10C), and the switching binder of SEQ ID NO. 2 (H2) (FIG. 10D), respectively. In an embodiment, the target antigen 20 may be HRP and the antibody 10 may be an anti-HRP antibody 10. The materials described above are exemplary and the present disclosure is not limited thereto.

In an embodiment, the amount of the isolated switching binder 130 was measured while increasing the concentration of the target antigen 20 in the analyte, and antibody 10 antigen-binding coefficient (K_{d}) was calculated using the amount of isolated switching binder 130. As for the antigen-binding coefficient (K_{d}), as the amount of HRP which is the target antigen 20 increased, the amount of fluorescence of the solution increased. In an embodiment, the antigen-binding coefficient (K_{d}) of the switching binder 100 may be 1.2 to 2.9 × 10⁻⁶ M. Preferably, the antigen-binding coefficient (K_{d}) of the switching binder of SEQ ID NO. 3 (L1) in FIG. 10A, the switching binder of SEQ ID NO. 4 (L2) in FIG. 10B, and the switching binder of SEQ ID NO. 2 (H2) in FIG. 10D may have values of 8.9 × 10⁻⁷ M, 1.2 × 10⁻⁶ M, and 2.9 × 10⁻⁶ M, respectively. Through this, unlike the method of FIG. 4Aof measuring the antigen-binding coefficient (K_{d}) after binding the antibody 10 to the solid support, the kit provided in the present disclosure provides a method for measuring the antigen-binding coefficient (K_{d}) when an antibody binds an antigen in a solution, and thus the antigen-binding coefficient (K_{d}) of the antibody in solution can be measured using the switching binder 100 that reversibly binds to the antigen-binding portion of the antibody.

The present disclosure described above is not limited to the above-described embodiments and examples and the accompanying drawings, and it will be apparent to those of ordinary skill in the art to which the present disclosure pertains that various substitutions, modifications, and changes can be made within the scope of the present disclosure without departing from the technical spirit of the present disclosure.

## Claims

1. A switching binder that reversibly binds to at least a portion of a framework region (FR) of an antibody and is separated from the framework region when a target antigen that specifically reacts with the antibody binds to the antibody.

2. The switching binder of claim 1, wherein the switching binder binds to the framework region by a three-dimensional structure of the antibody.

3. The switching binder of claim 1, wherein the switching binder comprises a moiety capable of binding to at least one framework region and a moiety capable of binding to at least one complementarity determining region (CDR).

4. A switching binder comprising an amino acid sequence selected from a framework region of a heavy chain constituting an antigen-binding portion of an antibody,
wherein the amino acid sequence contains a first antibody-binding portion reversibly bound to a framework region of a light chain constituting the antigen-binding portion together with the heavy chain.

5. A switching binder comprising an amino acid sequence selected from a framework region of a light chain constituting an antigen-binding portion of an antibody,
wherein the amino acid sequence contains a second antibody-binding portion reversibly bound to a framework region of a heavy chain constituting the antigen-binding portion together with the light chain.

6. The switching binder of claim 4 or 5, wherein the amino acid sequence further reversibly binds to a complementarity determining region adjacent to the framework region of the heavy chain or the light chain.

7. The switching binder of claim 4 or 5, wherein the switching binder is selected from the group consisting of a peptide, a protein, an aptamer, a nanobody, a phage display, a yeast display, and an antibody.

8. The switching binder of claim 4 or 5, wherein a portion of the amino acid of the switching binder is substituted with an inorganic material.

9. The switching binder of claim 4, wherein the first antibody-binding portion comprises at least one amino acid residue of -Y-, -I-, -W-, and -Q-.

10. The switching binder of claim 5, wherein the second antibody-binding portion comprises at least one amino acid residue of -Y-, -Q-, -P-, -L-, and -F-.

11. The switching binder of claim 4 or 5, wherein the switching binder comprises any one of the amino acid sequences of
SEQ ID NO. 1: S-Y-W-I-H-W-V-K-Q-R-P-G-Q-G-L-E-W-I-G-E,
SEQ ID NO. 2: V-Y-Y-C-A-R-E-P-T-G-T-G-I-Y-F-D-V-W-G-K,
SEQ ID NO. 3: T-Y-L-E-W-Y-P-Q-K-P-G-Q-S-P-K-L-L-I-Y-K, and
SEQ ID NO. 4: V-Y-Y-C-F-Q-G-S-H-V-P-F-T-K.

12. The switching binder of claim 4, wherein the first antibody-binding portion comprises an amino acid residue that binds to the framework region of the light chain by at least one selected from electrostatic interaction, hydrogen bond, van der Waals force, hydrophobic interaction, and a combination thereof.

13. The switching binder of claim 5, wherein the second antibody-binding portion comprises an amino acid residue that binds to the framework region of the heavy chain by at least one selected from electrostatic interaction, hydrogen bond, van der Waals force, hydrophobic interaction, and a combination thereof.

14. The switching binder of claim 12, wherein the binding strength of the antibody-binding site is regulated by amino acid modulation of the first antibody-binding portion.

15. The switching binder of claim 13, wherein the binding strength of the antibody-binding site is regulated by amino acid modulation of the second antibody-binding portion.

16. The switching binder of claim 12 or 13, wherein a bond of a side chain functional group or a polar site of a peptide bond between the switching binder and the framework region is performed by the hydrogen bond.

17. The switching binder of claim 4 or 5, wherein when the switching binder is bound to the antigen-binding site of the antibody, the switching binder is separated from the antibody by binding a target antigen that specifically reacts with the antibody to the antibody.

18. The switching binder of claim 4 or 5, wherein the antibody-binding portion of the switching binder is bound to the antigen-binding site of the heavy chain or the light chain within a range of 4 Å to 8 Å.

19. The switching binder of claim 4 or 5, wherein the switching binder is labeled with a natural or artificial labeling material.

20. The switching binder of claim 4 or 5, wherein the switching binder binds to a human antibody, a non-human antibody, a humanized non-human antibody, or a combination thereof, and the antibody can be bound to the target antigen in a state where the switching binder is bound to the antibody.

21. A switching binder comprising: a first antibody-binding portion or a second antibody-binding portion that is reversibly and specifically bound to at least some of framework regions (FRs) of an antibody; and
a binding strength regulating portion for adjusting a binding strength of the first antibody-binding portion or the second antibody-binding portion to the framework regions such that the antibody-binding portion is separated from the framework regions when a target antigen that specifically reacts with the antibody binds to the antibody.

22. The switching binder of claim 21, wherein the binding strength regulating portion modulates a position of the first antibody-binding portion or the second antibody-binding portion in the switching binder, or a three-dimensional shape of the switching binder, to adjust the binding strength of the antibody-binding portion to the antibody.

23. The switching binder of claim 21, wherein the binding strength regulating portion comprises at least one amino acid sequence selected from -S-Y-W-I-H-W-V-K-, -R-P-G-Q-G-L-E-, -I-G-E-, -V-Y-, -C-A-R-E-P-T-G-T-G-, -Y-F-D-V-, -G-K-, -T-Y-L-E-W-Y-, -K-P-G-Q-S-, -K-, L-I-Y-K-, -C-F-Q-G-S-H-V-P-, and -T-K-.

24. The switching binder of claim 21, comprising the first antibody-binding portion or the second antibody-binding portion and the binding strength regulating portion as a unit structure, such that the antibody-binding portion and the binding strength regulating portion are alternately coupled to each other consecutively.

25. A pharmaceutical composition for disease screening, diagnosis, prophylaxis, or treatment integration, comprising the switching binder of claim 4 or 5.

26. The pharmaceutical composition of claim 25, wherein the switching binder is selected from the group consisting of a peptide, a protein, an aptamer, a nanobody, a phage display, a yeast display, and an antibody.

27. The pharmaceutical composition of claim 25, wherein the switching binder comprises any one of the amino acid sequences of
SEQ ID NO. 1: S-Y-W-I-H-W-V-K-Q-R-P-G-Q-G-L-E-W-I-G-E,
SEQ ID NO. 2: V-Y-Y-C-A-R-E-P-T-G-T-G-I-Y-F-D-V-W-G-K,
SEQ ID NO. 3: T-Y-L-E-W-Y-P-Q-K-P-G-Q-S-P-K-L-L-I-Y-K, and
SEQ ID NO. 4: V-Y-Y-C-F-Q-G-S-H-V-P-F-T-K.

28. A test kit comprising the switching binder of claim 4 or 5,
wherein the switching binder is labeled with a natural or artificial labeling material, and the switching binder measures a binding strength of an antigen to an antibody by measuring an amount of the switching binder bound to and dissociated from framework regions.

29. The test kit of claim 28, wherein the switching binder is selected from the group consisting of a peptide, a protein, an aptamer, a nanobody, a phage display, a yeast display, and an antibody.

30. The test kit of claim 28, wherein the binding strength is measured by calculating an antigen-binding coefficient (K_{d}) of the antibody.

31. The test kit of claim 29, wherein the antigen-binding coefficient (K_{d}) is in a range of 0.7 × 10⁻⁶ M to 3.4 × 10⁻⁶ M.

32. The test kit of claim 29, wherein the test kit measures the antigen-binding coefficient (K_{d}) when the antibody binds to the antigen in a solution in which the antibody is not immobilized.

33. A test kit comprising the switching binder of claim 4 or 5,
wherein the switching binder is labeled with a natural or artificial labeling material, and the switching binder identifies and quantifies an antibody and a target antigen by measuring an amount of the switching binder bound to and dissociated from framework regions.

34. The test kit of claim 33, wherein the test kit is a kit for immunoassay,
wherein the kit for immunoassay comprises a luminex assay, a protein microarray assay, an ELISA assay, an immuno-PCR assay, a capture-ELISA assay, an ELISPOT assay, a radioimmunoassay (RIA), a lateral flow immunoassay (LFIA), a flow-through immunoassay (FTIA), a protein microfluidic immunoassay, a protein capillary electrophoresis (CE) assay,
an electrochemical biosensor, a radioimmunoprecipitation assay, an immunoprecipitation assay, an immunohistochemical staining assay, an Ouchterlony immunodiffusion assay, an inhibition or competition assay, a sandwich assay, a flow cytometry, an immunoelectrophoretic assay, a tissue immunostaining assay, a complement fixation assay, a FACS assay, a protein chip assay, an immunofluorescence staining assay, and an immunoaffinity purification assay kit.

35. An assay method using a switching binder, comprising the steps of:
selecting an amino acid sequence of the switching binder from framework regions constituting an antigen-binding site of an antibody;
pretreating to reversibly bind to at least a portion of the framework regions of the antibody by providing the switching binder to the antibody that specifically binds to a target antigen;
providing an analyte that requires determination of whether the target antigen is included in the result of the pretreatment step; and
analyzing to identify or quantify the target antigen in the analyte by quantifying at least one of a residual amount of the switching binder bound to the framework regions or the switching binder separated from the framework regions.

36. The assay method of claim 35, wherein the switching binder is selected from the group consisting of a peptide, a protein, an aptamer, a nanobody, a phage display, a yeast display, and an antibody.

37. The assay method of claim 35, wherein the antibody is immobilized on a support selected from a substrate, a bead, a fiber, a polymer structure, or a combination thereof.

38. The assay method of claim 35, wherein the antibody is not immobilized but dissolved in a solution.

39. The assay method of claim 35, wherein the pretreatment step comprises
providing the switching binder to the antibody to obtain a mixed solution containing a first conjugate of the antibody and the switching binder, and a switching binder not bound to the antibody, and
removing the switching binder not bound to the antibody from the mixed solution.

40. The assay method of claim 35, wherein the switching binder is labeled with a natural or artificial labeling material, and in the analysis step, the analysis of the target antigen is performed by measuring the labeling reaction.

41. The assay method of claim 35, further comprising the steps of:
separating the switching binder separated from the antibody, and a second conjugate of the antibody and the target antigen; and
identifying or quantifying the separated switching binder.

42. A method for preparing a switching binder that reversibly and specifically binds to a framework region (FR) of an antibody and is separated from the framework region when a target antigen that specifically reacts with the antibody is bound, the method comprising the steps of:
identifying an amino acid sequence in which the framework region of a heavy chain and a light chain of the antibody self-bind with each other;
obtaining a hydrophobicity index of the amino acid sequence; and
comparing the hydrophobicity index such that the switching binder comprising an amino acid sequence of a portion having the same property between the amino acid sequences of the framework regions constituting an antigen-binding site of the heavy chain and the light chain is selected.

43. The method of claim 35, wherein the amino acid sequence comprises a complementarity determining region adjacent to the framework region.

44. The method of claim 35, wherein the switching binder is selected from the group consisting of a peptide, a protein, an aptamer, a nanobody, a phage display, a yeast display, and an antibody.

45. The method of claim 35, wherein the amino acid sequence is bound by at least one selected from electrostatic interaction, hydrogen bond, van der Waals force, hydrophobic interaction, and a combination thereof.
